# EUROPEAN PATENT APPLICATION

(11) **EP 2 787 004 A1**
(43) Date of publication of application: **08.10.2014**
(21) Application number: 13162130.2
(22) Date of filing: 03.04.2013
(51) Int. Cl.: C07K 14/415, C12N 15/82, C12Q 1/68

(54) **A method for predicting a "mantled" phenotype in oil palm plants and minimizing the percentage of oil palm plants with a "mantled" phenotype in a culture, and polynucleotide to be used therein**

(71) Applicant: Malaysian Palm Oil Board, 43000 Kajang, Selangor (MY)
(72) Inventor: Ong Abdullah, Meilina, 70300 Seremban, Negeri Sembilan (MY); Ooi, Siew Eng, 43300 Seri Kembangan, Selangor (MY); Ishak, Feshah, 43000 Kajang, Selangor (MY); Smulders, Marinus Johannes Maria, 7339 KA Ugchelen (NL); Esselink, Gerhard, 6603 KE Wijchen (NL); Van der Linden, Cornelis Gerardus, 6721 CK Bennekom (NL)
(74) Representative: Bras, Pieter

(57) **Abstract**

The present invention provides a method for predicting a mantled phenotype in oil palm plants, comprising analyzing DNA material from tissue samples oil palm plants for the degree of methylation in a genomic region contained in the genome of the oil palm plant. The method of the invention can be applied for predicting a mantled phenotype in oil palm plants, in particular for predicting in young oil palms the development of a mantled phenotype in a later stage of their life cycle. Method of the invention can also be applied to identify cultures with a high risk of producing ramets that will develop a mantled phenotype. The invention thus also relates to a method to minimize the percentage of oil palm plants with a mantled phenotype in a culture. The invention also provides an isolated polynucleotide for use as a biomarker in a method to predict a mantled phenotype in oil palms.

## Description

### Field of the invention

The present invention relates to an isolated polynucleotide or a nucleic acid construct comprising said polynucleotide and its use for predicting the mantled phenotype in oil palm plants. The invention also relates to a method for predicting a mantled phenotype in an oil palm plant, in particular for predicting in young oil palms, which do not yet develop flowers, the development of a mantled phenotype in a later stage of their life. Furthermore, the invention relates to a method to minimize the percentage of oil palm plants with a mantled phenotype in a culture.

### Background of the invention

As the world's population continues to increase, so does the global food demand. This drives the global demand for vegetable oils. Palm oil contributes 57% of the world's export of vegetable oils (Oil World Annual 2009). Southeast Asia, particularly Indonesia and Malaysia, is now the largest producer of palm oil. With the added versatility for biofuel generation, the demand for palm oil will continue to increase. The oil palm is also the most productive oil crop in the world. At an average oil yield of 3.3 t/ha compared to soybean which yields 0.4 t/ha and rapeseed with a yield of 1.3 t/ha, the oil palm uses land more efficiently than any of the other vegetable oil crops (Hardter and Fairhurst 2003).

The most cultivated type of oil palm for oil yield is *Elaeis guineensis* and in particular the *tenera,* an F1 hybrid produced by crossing thickshelled *dura* with shell-less *pisifera.* The F1 hybrids are in fact populations, as both parents are highly heterozygous. Breeding programs are implemented in major oil palm plantations to improve planting material with desired traits, which include higher bunch yield and oil extraction rate, rapid crown expansion, and small height increment (Breure 2003). High yielding and dwarf palms with a potential oil yield of 7.7 t/ha/yr compared to the normal *tenera* palms with 5-6 t/ha/yr were developed by the Malaysian Palm Oil Board (MPOB) after an intense selection of the Nigerian germplasm collection in MPOB (Mohd Basri et al., 2004).

For more than 20 years, the Malaysian national average palm oil yield has been stagnating at 3.5 to 3.9 t/ha/yr. With increasing global demands for vegetable oils coupled with challenges in production efforts, there is a crucial need to improve productivity. One obvious route to accelerate and capture the maximum yield potential is to vegetatively propagate the superior genotypes selected from the F1 *tenera* families, or to vegetatively propagate the parents of crosses which produced such superior palms. As palms do not have axilliary meristems, these selected oil palm trees need to be vegetatively propagated through tissue culture. The original plant from which a plant is started is referred to as ortet and a vegetatively reproduced copy of a plant is called ramet.

Tissue culture of the oil palm was initiated in the 1970s (Jones 1974; Rabéchault and Martin 1976). Optimizations primarily in using low phytohormone medium have led to established protocols applied by the industry. Results from clonal trials of selected genotypes have realised a 30% increase in yield projection compared to the average of the F1 *tenera* seedlings (Corley et al., 1982; Hardon et al., 1987; Corley and Law 1997; Duval et al., 1997). The global production of clonal oil palm plantlets reported in 2007 was approximately 3 million (Rajanaidu et al., 2007). This translates to more than 20,000 hectares if planted at 148 palms/hectare.

Unfortunately, an abnormality phenomenon referred to as "mantling" was reported in the 1980s (Corley et al., 1986; Eeuwens et al. 2002). This abnormality affects the morphology of the flower, whereby supernumerary carpels are formed that lead to loss of flower bunches. The supernumerary carpels develop from the staminodes and stamens of the pistillate flowers. Also functional staminate flowers may be affected, in which case pseudocarpels develop (Adam et al., 2005). The mantling abnormality is only manifested when the palms start flowering at 1.5 to 2 years after field planting. Some palms revert to a normal flower phenotype later on, this is also referred as the revertant phenotype. These palms are referred to as revertants. A somaclonal variant displaying supernumerary carpels has also been observed in date palm which can revert to normal after several years (Cohen et al., 2004). Fine-tuning and optimization of the oil palm tissue culture protocol has led to lower mantling rates in clonal plantings. Laboratories have reported reduced mantling rates, with rates of about 5% with some as low as 1% (Maheran and Abu Zarin 1999; Soh et al., 2001). This is likely due to better management of cultures coupled with stringent culling at various stages along the process (Maheran et al., 1993; Wooi 1993; Simon et al., 1998), which does mean that the maximum multiplication rate is rather limited.

As mentioned above, the mantling phenotype is an abnormality affecting the morphology of the flower. The morphology of flowers is determined by the activity of a number of homeotic genes during flower development, most of which belong to the MADS-box family of transcription factors. These homeotic genes are conserved among dicotyledons, monocotyledons, and conifers (Purugganan et al., 1995; Weigel and Nilsson 1995; Tandre et al., 1998). The quartet model, that evolved from the classical ABC model, explains the combinatorial action of the organ identity proteins, mostly encoded by MADS-box genes (Theissen 2001; Sablowski 2010). Phenotypes resembling the mantled oil palm have been described in *Arabidopsis thaliana* and *Antirrhinum majus.* Most of these phenotypes are due to disruption of B-class MADS box gene expression, AP3/PI or DEF/GLO respectively, whereby the petals are transformed into sepals and stamens transformed into carpels (Coen and Meyerowitz 1991; Kang et al., 1998; Zahn et al., 2005). In rice, overexpression of OSMADS4, a putative B-type MADS-box gene, also resulted in the conversion of the lodicules to lemma-like structures and the third whorl stamens to carpel-like organs (Kang et al., 1998). The expression of oil palm orthologues of class B, C and E MADS-box genes was reduced in mantled flowers (Adam et al., 2007).

On the basis of several observations the mantling abnormality is most likely an epigenetic event, which means that heritable changes in gene expression or phenotype are caused by mechanisms other than changes in the underlying DNA sequence. The main evidence stems from the fact that the same abnormality occurs frequently (Syed Alwee et al. 2006; Smulders and De Klerk 2011), that there exist various degrees of penetration of the abnormality, and that abnormal palms can revert to a normal flower phenotype (Jaligot et al., 2000; Kubis et al., 2003; Syed Alwee et al. 2006; Jaligot et al., 2011). It has been reported that a low NAA:kinetin ratio increases the incidence of mantling in oil palm (Eeuwens et al., 2002). Kinetin has been demonstrated to cause extensive DNA hypomethylation while NAA causes hypermethylation instead (LoSchiavo et al., 1989; Arnholdt-Schmitt et al., 1991; Eeuwens et al., 2002). In accordance with these observations, global methylation levels in mantled ramets were also slightly lower than their normal counterparts (Jaligot et al., 2000). There is a strong reduction in methylation during tissue culture (callus and suspension cultures) which is normally recovered to near-normal methylation levels in the ramets, however these levels are still lower than that in the parental ortet (Kubis et al., 2003). It is possible that some genomic regions might be less methylated, while others are more methylated although overall methylation levels remain similar between ortet and regenerant.

The pattern of DNA methylation changes throughout the life cycle of a plant. During the *in vitro* phase, the overall methylation percentage is often reduced (Kaeppler and Phillips 1993). Cytosine methylation usually results in a loss of gene expression (Matzke and Matzke 1993; Finnegan et al., 1998; Luff et al., 1999), so a change in methylation can have similar consequences for the phenotype as a loss-of-function mutation. If errors occur in the restoration of 'normal' DNA methylation patterns during regeneration, these may lead to the repeated occurrence of changes in certain traits. Such epimutations may be more or less stable in regenerated plants and during clonal propagation, but not inherited to offspring (Kaeppler and Phillips 1993; Smulders et al., 1995; Kaeppler et al., 2000). For these reasons, DNA methylation is considered to be a potential mechanism by which gene expression in oil palm ramets may be changed after micropropagation.

As the basic cause for the development of a mantled phenotype in oil palms is still not known, tissue culturists are keen to have a screening tool for the early detection of the mantling abnormality to circumvent the risk of encountering mantling in field-planted clones. WO03/040297 describes a protein of the defensin family, for which the expression level is higher in callus producing a high percentage of mantled ramets. However, the expression level of this gene can only be applied as a molecular marker of somaclonal variation events associated with the mantled phenotype in the callus stage of tissue culture, and not later on.

Auyong et al. (2005) have investigated the possibility to predict mantled phenotypes by methylation differences in a MADS-box domain between tissue of "mantled" and normal oil palm. A screen for abnormal DNA methylation profiles in leaf tissue of flowering oil palm plants showed that polymorphisms appeared to be clonal-specific. Furthermore, polymorphisms identified in Auyong et al. (2005) only provided a means for epigenetic variations of off-type individuals in hindsight, because only tissue of flowering oil palm plants was used.

For these reasons, there is a need for a biomarker, which is broadly applicable to predict the mantled phenotype, in particular in early developmental stages, of oil palm plants.

The present invention provides such a biomarker. The present invention is based on the knowledge that methylation of DNA is a potential mechanism by which gene expression in oil palm ramets may be changed after micropropagation and the observation that methylation differences correlate with differences in the morphology of the flower. In particular, the invention is based on the observation that methylation differences in oil palm ramets can be correlated with the mantling abnormality phenomenon, whereby supernumerary carpels are formed that lead to a loss of flower bunches and thus economic losses through plant and oil losses.

Because it is known that the pattern of DNA methylation changes throughout the life cycle of a plant, especially in the *in vitro* phases of tissue culture processes, it was a surprise that the inventors found a biomarker that can be applied in a method based on DNA methylation differences for predicting a mantled phenotype in an oil palm plant, in particular for identifying cultures with a high risk of producing plants that will develop a mantled phenotype, and for predicting in young oil palms, which do not yet develop flowers, the development of a mantled phenotype in a later stage of their life. The invention thus provides a biomarker that may be used in various stages of the oil palm life cycle i.e. the callus stage, the shoot developing stage, in the nursery stage and in the field.

The present invention provides isolated polynucleotide sequences and their use as biomarkers in a method to predict the mantled phenotype in oil palm plants. Although the biomarkers are not applicable for all clones tested, it can be used in a broad set of clones to predict a mantled phenotype in oil palm plants, in particular for predicting in young oil palms the development of a mantled phenotype in a later stage of their life cycle. Because of this, isolated nucleotides of the invention can be applied to identify, in a stage in which oil palm plants have not yet developed flowers, cultures with a high risk of developing a mantled phenotype. The present invention also provides a method to predict a mantled phenotype in oil palm plants, comprising analyzing a tissue sample from oil palm plants for hypomethylation of a differentially methylated region. This method can be implemented in a strategy to minimize the percentage of oil palm plants with a mantled phenotype in a culture by excluding palms that are predicted to develop a mantled phenotype from further propagation. The ability to diagnose the mantled phenotype in early micropropagated plant material thus provides the oil palm industry with the means to prevent the present economic losses through plant and, hence, oil losses.

### Summary of the Invention

Nucleotide and amino acid sequences are referred to by a sequence identifier number (SEQ ID NO:). The SEQ ID NOs: correspond numerically to the sequence identifiers <400>1 (SEQ ID NO: 1), <400>2 (SEQ ID NO: 2), etc.

The present invention provides in one aspect an isolated polynucleotide selected from the group consisting of:
a) a polynucleotide derived from an oil palm plant having a nucleotide sequence that is at least 70% identical to the nucleotide sequence of SEQ ID NO: 1;
b) a fragment of polynucleotide a) comprising at least 10 contiguous nucleotides;
c) a polynucleotide complementary to polynucleotide a) or b) derived from an oil palm plant; and
d) a polynucleotide hybridising selectively with polynucleotide a), b) or c).

The present invention also provides a nucleic acid construct comprising the polynucleotide.

In another aspect the invention provides the use of the abovementioned polynucleotide or nucleic acid construct for predicting the mantled phenotype in oil palm plants at any age, in particular for predicting in young oil palm plants the development of a mantled phenotype in a later stage of their life cycle.

In still another aspect the invention provides a method for predicting a mantled phenotype in an oil palm plant, wherein DNA material, comprising the nucleotide sequence of the polynucleotide of a) a polynucleotide having a nucleotide sequence that is at least 70% identical to the nucleotide sequence of SEQ ID NO: 1; b) a fragment of polynucleotide a) comprising at least 10 contiguous nucleotides; or c) a polynucleotide complementary to polynucleotide a) or b), is isolated from said plant and the degree of methylation is analysed in a region of said DNA material having said nucleotide sequence. Such a method may be in particular applied for predicting in young oil palm plants the development of a mantled phenotype in a later stage of their life cycle, in order to exclude palms that are predicted to develop a mantled phenotype from further propagation.

The invention in particular provides a method for minimizing the percentage of oil palm plants with a mantled phenotype in a culture, wherein DNA material, comprising the nucleotide sequence of a) a polynucleotide having a nucleotide sequence that is at least 70% identical to the nucleotide sequence of SEQ ID NO: 1; b) a fragment of polynucleotide a) comprising at least 10 contiguous nucleotides; or c) a polynucleotide complementary to polynucleotide a) or b), is isolated from oil palm plants and analysed for the degree of methylation in a region of said DNA material having said nucleotide sequence, wherein plants wherein the DNA in said region is hypomethylated are removed from the culture, wherein said oil palm plants preferably are young plants, such as *in vitro* cultures.

In one embodiment, this method comprises the following subsequent steps:
i) isolating DNA material, comprising the nucleotide sequence of the polynucleotide of claim 1 a), b) or c), from a plurality of young plants of a plurality of oil palm clones;
ii) analysing for the degree of methylation in a region of said DNA having said nucleotide sequence,
iii) determining in which clones hypomethylation in said region is indicative for developing the mantled phenotype,
iv) selecting clones in which hypomethylation in said region is indicative for developing the mantled phenotype; and
v) maintaining in the culture the selected clones wherein less than 50% of the plants is hypomethylated in said region and wherein less than 50% of the plants develops a mantled phenotype, while removing the rest of the clones from the culture.

In such an embodiment preferably in step v) the selected clones wherein less than 5% of the plants is hypomethylated in said region and wherein less than 5% of the plants develops a mantled phenotype are maintained in the culture, while the rest of the clones are removed from the culture.

Optionally, this embodiment comprises the additional steps:
vi) repeating steps i) and ii) in young plants derived from the clones selected in step iv); and
vii) removing the plants which are hypomethylated in said region from the culture.

### Short description of the figures

Figure 1. The assay. MM-78-PCR products generated from a normal ramet (N) and an abnormal (mantled) ramet (A).
Figure 2. MM78-PCR products on field-planted ramets from MPOB. (A) Clone P164 ramets, 11 years old (1-21); N: P164 normal ramet; A: P164 abnormal ramet. (B) Clone 149 ramets, 10 years old (1-19); N: P149 normal ramet; A: P149 abnormal ramet. Arrows indicate the 300 bps band associated to mantled phenotype in the tested ramets. M: 100 bps ladder (Promega).

### Detailed description of the invention

The present invention provides isolated polynucleotides and their use in a method for predicting the mantled phenotype in oil palm plants. According to the invention, the isolated polynucleotides can be used in a method for minimizing the percentage of oil palm plants with a potential mantled phenotype in a culture or at the juvenile stage. The isolated polynucleotides may serve as biomarkers or as polynucleotide primer sequences that may be used to detect biomarkers. The isolated polynucleotide of the invention is selected from the group consisting of:
a) a polynucleotide derived from an oil palm plant having a nucleotide sequence that is at least 70% identical to the nucleotide sequence of SEQ ID NO: 1;
b) a fragment of polynucleotide a) comprising at least 10 contiguous nucleotides;
c) a polynucleotide complementary to polynucleotide a) or b) derived from an oil palm plant; and
d) a polynucleotide hybridising selectively with polynucleotide a), b) or c).

The terms "palm", "oil palm", "palm plant" and "oil palm plant" as used herein should be understood to refer to oil palm plants, which includes the species *Elaeis oleifera* and *Elaeis guineensis* including modified varieties or genetic variants thereof. Although other oil palms are included in the invention, the term "palm" relates in particular to oil palm plant types of the tenera type, which are obtained by crossing palms of the dura type with palms of the *pisifera* type. The term "oil palm plant" of the invention may refer to ortets and ramets, which may be in a juvenile stage, for instance in a nursery. The term oil palm plant may also refer to early micropropagated plant material or tissue culture.

The oil palm plant from which the tissue sample is to be taken is preferably a tissue culture propagated palm plant, a ramet. The tissue sample may be a sample from callus cell culture. Oil palm plant to be subjected to methods of the inventions may be of any age. The age of an oil palm plant is defined as the time passed after field planting. Samples of young palms may be taken at an age of the ramets of 3-4 years. However it is preferred to take samples from ramets at callus stage, the shoot developing stage or the juvenile stage (plantlets of until approximately 6 months old) in a nursery. A young oil palm should be considered as an oil palm plant that has not yet developed flowers or reached the stage wherein flowers are developed. Such a young oil palm plant may be in the callus stage, the shoot developing stage, in the juvenile stage or growing in the plantation. The term "young oil palms" also comprises cell from a callus cell culture or an embryogenic cell culture.

The biological markers of the invention consist of nucleic acid molecules. Reference herein to a "nucleic acid molecule" includes reference to DNA material, genomic DNA, DNA, cDNA, amplification product or PCR product. A nucleic acid molecule may also be referred to herein inter alia as, nucleotide sequence, polynucleotide sequence, oligonucleotide sequence, polynucleotide or oligonucleotide. Reference to a nucleic acid molecule includes genomic DNA. In this invention, the term biological marker or biomarker should be interpreted as a nucleotide sequence that may be specifically detected in case a genomic region of oil palm plants comprised in the isolated polynucleotide which is selected among a) a polynucleotide with at least 70% sequence identity to the nucleotide sequence of SEQ ID NO: 1, b) a fragment of polynucleotide a) comprising at least 10 contiguous nucleotides or c) a polynucleotide complementary to polynucleotide a) or b) has a particular methylation state. This may be a normal methylation state or a hypomethylation state, wherein a normal methylation state is indicative for an oil palm plant developing normal flowers and hypomethylation is indicative for an oil palm plant developing the mantled phenotype.

The polynucleotide mentioned under d) of the previous paragraph refers to a polynucleotide that hybridises selectively with the polynucleotides mentioned under a), b) and c). In this invention the polynucleotide mentioned under d) therefore particularly refers to a polynucleotide primer sequence in PCR procedures that can be applied in detection of the biomarker of the invention.

In some stage of the method of the invention the biological markers exist in the form of isolated nucleic acid molecules. By "isolated" is meant material that is substantially or essentially free from components that normally accompany it in its native state. An example of such a molecule is an isolated nucleic acid molecule, which has been purified from the sequences which flank it in a naturally occurring state, e.g. a DNA fragment which has been removed from the sequences which are normally adjacent to the fragment. An isolated nucleic acid molecule may be obtained by genomic DNA isolation, digestion by endonuclease enzymes, PCR, DNA extraction, DNA purification or any method that will be known by the person skilled in the art. Isolated nucleic acid molecules may be obtained from any nucleic acid source, especially from biological samples or tissue samples. The isolated nucleic acid molecule may be derived from genomic DNA or directly from biological samples from a palm plant. The term "biological samples" in the context of the invention is defined to include any sample derived from any part of an oil palm plant. In the context of the invention, biological samples include tissue samples that may be derived from any material of oil palms. Preferably, tissue samples are derived from young leaves. Tissue samples may be obtained from ramets or ortets still existing in the field. Tissue samples may also be taken from oil palms at juvenile stage. Tissue samples may be stored in a freezer before analysis according to one of the methods of the invention.

The term "nucleic acid molecule" of the present invention encompasses derivatives, including mutants and homologues thereto. The term "nucleotide sequence" or "sequence of nucleotides" of the present invention encompasses derivatives, including the complementary sequence thereof.

By derivative is meant any single or multiple nucleotide deletions, additions or substitutions as well as mutants, fragments, portions or parts of the isolated nucleic acid molecule. All such deletions, additions, extensions, substitutions as well as mutants, fragments, portions, or parts are encompassed by the term "derivative". Therefore, the present invention also provides a nucleic acid construct comprising the abovementioned isolated polynucleotide. Useful derivatives include alterations to the 5' end portion of the polynucleotide sequence or the 3' end portion or a nucleotide sequence spanning the 5' and 3' portions. A derivative of the polynucleotide of the invention includes a polynucleotide hybridising selectively under stringent conditions with a) a polynucleotide with at least 70% sequence identity to the nucleotide sequence of SEQ ID NO: 1, b) a fragment of polynucleotide a) comprising at least 10 contiguous nucleotides or c) a polynucleotide complementary to polynucleotide a) or b). A derivative of the nucleotide sequences of the invention also conveniently includes a nucleotide sequence having less than 100% sequence identity with an isolated polynucleotide which is selected from the group consisting of a) a polynucleotide with at least 70% sequence identity to the nucleotide sequence of SEQ ID NO: 1, b) a fragment of polynucleotide a) comprising at least 10 contiguous nucleotides or c) a polynucleotide complementary to polynucleotide a) or b), but which is capable of hybridizing thereto under low stringency conditions at 42°C. A derivative of the nucleotide sequence set forth in SEQ ID NO: 1 preferably shows between 70 and 100% sequence identity to the nucleotide sequences set forth in SEQ ID NO: 1, such as 75 %, 80%, 85%, 90% or 95% sequence identity.

Sequence identity can be determined by alignment of two nucleotide sequences using global or local alignment algorithms. Sequences may then be referred to as "substantially identical' when they (when optimally aligned by for example the programs GAP of BESTFIT using default parameters) share at least a certain minimal percentage of sequence identity (in this case 70%). GAP uses the Needleman and Wunsch global alignment algorithm to align two sequences over their entire length, maximizing the number of matches and minimizing the number of gaps. Generally, the GAP default parameters are used, with a gap creation penalty = 50 for nucleotides and a gap extension penalty = 3 for nucleotides. For nucleotides the default gap scoring matrix used in nwsgapdna (Henikoff & Henikoff, 1992, PNAS 89, 915-919). Sequence alignments and scores for percentage identity may be determined using computer programs such as NEEDLE from the EMBOSS package. Sequence identity may also be determined by searching against databases such as FASTA, BLAST etc. The skilled artisan will acknowledge that different parameters will yield slightly different results but that the overall percentage identity of two sequences will not be significantly altered when using different algorithms.

In the sequences of polynucleotides of the invention, the IUPAC (International Union of Pure and Applied Chemistry) nucleic acid notation is applied. Guanine, Cytosine, Adenosine and Thymine are represented by the letters G, C, A and T, respectively. Some of the sequences of the invention contain degenerate base symbols. This is in particular the case for degenerate primers. Degenerate base symbols are an IUPAC representation for a position on a DNA sequence that can be have multiple possible alternatives. Table 1 shows these symbols and the bases they represent.

**Table 1. Degenerate base symbols**

| **Symbol** | **Bases represented** |
|---|---|
| w | A or T |
| s | C or G |
| m | A or C |
| k | G or T |
| r | A or G |
| y | C or T |
| b | C or G or T |
| d | A or G or T |
| h | A or C or T |
| v | A or C or G |
| n | A or C or G or T |

The length of the nucleotide sequence of the isolated polynucleotide of the invention will be dependent on the method used to analyze DNA material from a tissue sample from one or more oil palm plants for the degree of methylation. Said length may be dependent on the restriction endonucleases used in the method, the adapters used to ligate digestion products, the primers used in PCR methods suitable for the invention, etc.

The isolated polynucleotide of the invention comprises between 10 and 4882 consecutive nucleotides. The size of the polynucleotide depends on the role it fulfills in the method of the invention and the parameters used in the method of the invention. When the isolated polynucleotide is destined to hybridise selectively to a region of interest, the isolated polynucleotide of the invention preferentially comprises between 10 and 50 nucleotides, because polynucleotides of such size may conveniently be used as primers in a PCR procedure.

When the isolated polynucleotide of the invention is to be detected as a biomarker or as a part of a biomarker, the isolated polynucleotide can be of any size that can be conveniently detected via detection methods that are known in the art. The size of the detected biomarker may depend on the restriction enzyme(s) used, and on the position where the polynucleotide primers hybridise with the sequence of interest. Also the adapters that may be used influence the size of the biomarker. Depending on the adapter sequence, which may for instance be a nucleic acid vector, and primers used for PCR methods, the biomarker to be detected may as well be larger in size than 4882 bps.

When a PCR method is applied to amplify a biomarker, the method of the invention will be of longer duration if the area of interest to be amplified is large, because amplification will take a longer time. On the other hand, if the biomarker is very small in size, detection of it may be difficult. Therefore, the preferred size of biomarker of the invention is between about 200 and 1000 base pairs, although shorter or longer biomarkers are also applicable.

The present invention also provides the use of the abovementioned isolated polynucleotide or a nucleic acid construct comprising said polynucleotide in a method for predicting the mantled phenotype in oil palm plants. In this method a method an isolated polynucleotide selected among a) a polynucleotide with at least 70% sequence identity to the nucleotide sequence of SEQ ID NO: 1, b) a fragment of polynucleotide a) comprising at least 10 contiguous nucleotides and c) a polynucleotide complementary to polynucleotide a) or b); derived from DNA material of a tissue sample from an oil palm plant, in particular of a young oil palm plant is analysed for the degree of methylation. In this method hypomethylation is indicative for the mantled phenotype or the development thereof.

The inventors observed that the prediction provided by the method of the invention is not applicable for all clones. However, the method is applicable to a broad range of clones. Therefore the prediction is sufficient to provide a tool for lowering the percentage of young oil palm plants developing a mantled phenotype in a population. Because of the long maturing time required for an oil palm plant, removing plants that show false positive results with respect to hypomethylation at a young age is more attractive than allowing these plants to grow to their flowering stage and occupy space in the plantation for years.

An attractive alternative is to optimize the efficiency of the method in the long run by first selecting clones in which the biomarker of the invention is applicable. To select these clones, an isolated polynucleotide selected among a) a polynucleotide with at least 70% sequence identity to the nucleotide sequence of SEQ ID NO: 1, b) a fragment of polynucleotide a) comprising at least 10 contiguous nucleotides and c) a polynucleotide complementary to polynucleotide a) or b); derived from DNA material of a tissue sample from a number of young plants of one or more *E. guineensis* clones is analysed for hypomethylation. After this it is determined which plants actually develop the mantled phenotype. Subsequently it is determined in which clones (i.e. groups of plants representing one clone) hypomethylation is indicative for developing the mantled phenotype.

These clones are selected and subsequently micropropagated in a culture and the method can be used to predict development of a mantled phenotype in a later stage by analysing hypomethylation of these clones as described above. Preferably, clones are selected for micropropagation or cultivation when in less than 50% of the plants of a given clonal pool, hypomethylation is found and when this corresponds to a percentage of less than 50 % of the plants developing a mantled phenotype and accordingly when in more than 50% of the plants of a given clonal pool normal methylation is found and when this corresponds to a percentage of more than 50 % of the plants developing a normal phenotype. Preferred clones show close correlation (with a Pearson's correlation coefficient r of more than 0.7, preferably more than 0.8) between hypomethylation and development of mantled phenotype. It will be acknowledged by the person skilled in the art that the percentage of mantled plants (both predicted and in the plantation) is as low as possible to maximize cultivation efficiency. In a preferred situation such a percentage is lower than 5 %.

The method for predicting a mantled phenotype in an oil palm plant comprises analysis of the polynucleotide(s) of the invention for the degree of methylation therein wherein hypomethylation in selected clones is indicative for the mantled phenotype. Accordingly, the method of the invention can also be applied to predict a normal phenotype in an oil palm plant based on normal methylation of the polynucleotide(s) of the invention, wherein normal methylation or lack of hypomethylation is indicative for a normal phenotype. Thus, whether an embodiment of the method leads to normal methylation state biomarkers or hypomethylation biomarkers, either way the method enables prediction of the mantled phenotype. The polynucleotide of the invention isolated from an oil palm may be considered hypomethylated if the percentage of possible methylation sites of said polynucleotide that are not methylated is higher than the average percentage in oil palms of these possible methylation sites in a genomic area corresponding to the sequence of the polynucleotide of the invention.

The present invention thus provides methods that are based on the finding of an association between methylation of DNA and development of a "mantled" phenotype in oil palms. Furthermore the present invention provides methods, and nucleic acid sequences that are based on the finding that hypomethylation in an isolated polynucleotide selected among a) a polynucleotide with at least 70% sequence identity to the nucleotide sequence of SEQ ID NO: 1, b) a fragment of polynucleotide a) comprising at least 10 contiguous nucleotides and c) a polynucleotide complementary to polynucleotide a) or b), is correlated with the development of a mantled phenotype in oil palms.

Accordingly, methods of the invention analyze an isolated polynucleotide selected among a) a polynucleotide with at least 70% sequence identity to the nucleotide sequence of SEQ ID NO: 1, b) a fragment of polynucleotide a) comprising at least 10 contiguous nucleotides and c) a polynucleotide complementary to polynucleotide a) or b); to identify an increased risk of developing a mantled phenotype in an oil palm.

The invention provides a method for predicting a mantled phenotype in oil palms, comprising the following steps:
a) providing a tissue sample from an oil palm plant,
b) detecting a polynucleotide biomarker for the mantled phenotype contained in the polynucleotide of the invention.

The invention also provides a method for predicting in young oil palms the development of a mantled phenotype in a later stage of their life cycle, comprising the following steps:
a) providing a tissue sample from an oil palm plant,
b) detecting the polynucleotide biological marker for the mantled phenotype contained in the polynucleotide of the invention.

The method of the invention may be applied to identify, in a stage in which the oil palms have not yet developed flowers, cultures with a high risk of developing mantled phenotype, by providing tissue samples from cultures of nursery stage ramets, detecting the biological marker for the mantled phenotype contained in the isolated polynucleotide of the invention, comparing the percentages of presence of the biological marker for the mantled phenotype contained in the isolated polynucleotide of the invention, selecting the cultures with high frequencies of the biological marker for the mantled phenotype contained in the isolated polynucleotide of the invention as cultures with a high risk of developing mantled phenotype and removing cultures with a high risk of developing the mantled phenotype.

The sequence set forth in SEQ ID NO: 1 contains various CG or CNG sequences that are possible sites of cytosine methylation. Consequently, an area in the oil palm's genome corresponding to the sequence set forth in SEQ ID NO: 1 or a sequence that has at least 70% sequence identity thereto may be considered hypomethylated if the percentage of these methylation sites that are not methylated is higher than the average percentage in oil palms of these possible methylation sites in a genomic area corresponding to SEQ ID NO: 1 or a sequence that has at least 70% sequence identity thereto.

In particular CCGG, CGCG, CCGC and GCGC sequences can be recognized as restriction sites by the methylation sensitive restriction enzymes MspI/HpaII, BstUI, AciI and HinP1, respectively. Therefore, the isolated polynucleotide of the invention may be considered hypomethylated if at least one of the CCGG, CGCG, CCGC and GCGC sequences comprised in said polynucleotide is not methylated. The isolated polynucleotide of the invention may therefore be considered hypomethylated if at least one of the MspI/HpaII, BstUI, AciI and HinP1 restriction endonuclease restriction enzyme recognition sites comprised in said polynucleotide is not methylated.

The isolated polynucleotide of the invention may also be considered hypomethylated if the CCGG sequence corresponding to positions 2710-2713 of the sequence of SEQ ID NO: 1 or, after alignment to SEQ ID NO: 1, to the corresponding site in a sequence having at least 70% sequence identity thereto is not methylated. In a particular embodiment analysis for hypomethylation of this particular site may thus be used as a biomarker predicting the mantled phenotype in oil palm plants and consequently in a method for minimizing the percentage of mantled oil palm plants with a mantled phenotype in a culture.

The degree of methylation may be analyzed by contacting DNA material from the tissue sample with one or more methylation-sensitive restriction endonucleases, said one or more endonucleases cleaving specifically methylated recognition nucleotide sequences, which leads to methylation state specific digestion products.

The method of the invention may comprise methylation selective pretreatment of DNA of the oil palm. Such selective pretreatment may in particular be carried out using restriction endonucleases or restriction enzymes. In particular, methods of the invention comprise the digestion of genomic DNA of oil palm tissue with methylation sensitive restriction enzymes. Methylation sensitive restriction enzymes may be for instance MspJI, LpnPI, FspEI, DpnI, DpnII, McrBC, although other methylation sensitive restriction enzymes may be applicable as well.

In the method of the invention, digestion of genomic DNA of oil palm tissue with methylation sensitive isoschizomer enzymes can be applied. Isoschizomers are pairs of restriction enzymes that are specific to the same recognition sequence. In some cases, only one of a pair of isoschizomers is able to recognize the methylated as well as the unmethylated form of a restriction site. The other restriction enzyme, however is able to recognize only the unmethylated form of the restriction site.

In a preferred embodiment of the invention genomic DNA derived from tissue samples is digested with the methylation sensitive restriction enzyme HpaII. In another preferred embodiment of the invention, isoschizomer enzymes are MspI and HpaII. These enzymes both recognize the sequence 5'-CCGG-3' when it is unmethylated. However, when the internal C of the sequence is methylated, only MspI can recognize both the forms while HpaII cannot. Also other methylation sensitive isoschizomer enzymes may be used in methods of the invention. In another preferred embodiment of the invention the method of the invention genomic DNA derived from tissue samples is digested with the methylation sensitive restriction enzyme HpaII.

Digestion with restriction enzymes may be carried out using any suitable protocol supplied by the manufacturer of restriction enzymes (e.g. New England Biolabs, Fermentas etc.), any existing protocol or any variations and alterations thereto as will be understood by the person skilled in the art.

The present invention provides a method wherein a hypomethylation-specific digestion product is detected, which employs amplification by PCR of a polynucleotide selected from the group consisting of a) a polynucleotide with at least 70% sequence identity to the nucleotide sequence of SEQ ID NO: 1, b) a fragment of polynucleotide a) comprising at least 10 contiguous nucleotides and c) a polynucleotide complementary to polynucleotide a) or b) using a set of primers comprising at least one primer capable of hybridising selectively with polynucleotide a), b) or c) , wherein hypomethylation of the DNA material comprised in said sequences before contacting it with said one or more methylation-sensitive restriction endonucleases leads to differences in size of amplification products when compared to normally methylated polynucleotide sequences equivalent to polynucleotides a), b) or c). Said differences in size of amplification products are indicative of the methylation state of the tissue sample in a broad range of clones of the oil palm plant and this can be used to predict the mantled phenotype in selected clones of the oil palm plant in order to exclude palms that are predicted to develop a mantled phenotype from further propagation, and consequently to minimize the percentage of oil palm plants with a mantled phenotype in a culture.

The digestion products or amplification products may be ligated with adapters after digestion of the tissue sample with one or more methylation-sensitive restriction endonucleases, the adapters being nucleotide sequences. For this purpose genomic DNA derived from tissue samples may be digested with a methylation sensitive restriction enzyme, followed by ligation of the digestion products with adapters. Adapters may be any suitable nucleic acid molecule such as a DNA vector, one or more PCR primers or any suitable oligonucleotide or oligonucleotides. In a preferred embodiment adapters are comprised of double stranded adapters to which nucleotide sequences such as SEQ ID NO: 2 and/or SEQ ID NO: 3 may be able to hybridize. Ligation may be carried out using any suitable ligase, such as T4 ligase, while applying any suitable ligation method as will be understood by the person skilled in the art.

In this method genomic DNA may first be digested with a methylation sensitive enzyme or a set of methylation sensitive enzymes, for instance a set of methylation sensitive isoschizomer enzymes, in order to obtain a set of DNA fragments that may show variations between DNA samples depending on the state of methylation. The resulting digestion mix may be ligated with adapters. The ligation mix may then be subjected one or more PCR amplification steps.

In a PCR method of the invention, the set of primers comprises at least one primer hybridising selectively with a) a polynucleotide with at least 70% sequence identity to the nucleotide sequence of SEQ ID NO: 1, b) a fragment of polynucleotide a) comprising at least 10 contiguous nucleotides; or c) a polynucleotide complementary to polynucleotide a) or b), as well as at least one primer that is able to hybridize to the nucleotide sequence of at least one adapter.

In one embodiment of the invention, the set of primers comprises at least one primer hybridising selectively under stringent conditions with a) a polynucleotide with at least 70% sequence identity to the nucleotide sequence of SEQ ID NO: 1, b) a fragment of polynucleotide a) comprising at least 10 contiguous nucleotides; or c) a polynucleotide complementary to polynucleotide a) or b), as well as at least one primer that is able to hybridize to the nucleotide sequence of at least one adapter.

Alternatively, the set of primers comprises at least one primer hybridising selectively under low stringency conditions with a) a polynucleotide with at least 70% sequence identity to the nucleotide sequence of SEQ ID NO: 1, b) a fragment of polynucleotide a) comprising at least 10 contiguous nucleotides; or c) a polynucleotide complementary to polynucleotide a) or b). In such an embodiment low stringency PCR permits the identification of differentially methylated areas.

Stringent hybidisation conditions can be used to identify nucleotide sequences, which are substantially identical to a given nucleotide sequence. Stringent conditions are sequence dependent and will be different in different circumstances. Generally stringent conditions are selected to be about 5°C lower than the thermal melting point Tₘ for the specific sequences at a defined ionic strength and pH. The Tₘ is the temperature (under defined ionic strength and pH) at which 50% of the target sequence hybridizes to a perfectly matched probe. Typically stringent conditions will be chosen in which the salt concentration is about 0.02 molar at pH 7 and the temperature is at least 60°C. Lowering the salt concentration and/or increasing the temperature increases stringency.

Any suitable PCR amplification program, any suitable PCR device, any suitable PCR buffer, any suitable DNA polymerase and any suitable buffer combination may be used as will be understood by the person skilled in the art. Primers used for these PCR amplification steps may be designed based on the sequence of SEQ ID NO: 1 and surrounding genomic regions thereof. Primers may also be designed based on adapter sequences. The person skilled in the art will recognize that many primers consisting of different nucleotide sequences and many primer combinations will be suitable for PCR amplification of the biological marker of the invention. Such primers may be degenerate primers. Degenerate primers are mixtures of similar, but not identical primers.

Primers may be labeled to facilitate detection of PCR products. Such labels include but are not limited to radioactive labels, such as ³³P or ³²P, fluorescent labels, enzymatic labels, biotin etc.

For analysis of the degree of methylation, the DNA material may be subjected to a procedure selected from the group comprising:
a) chemical modification with bisulfite;
b) affinity-based isolation of methylated DNA;
c) amplification by PCR; and
d) treatment with methylation-sensitive restiction enzymes; or a combination thereof.

In one preferred embodiment of the invention, treatment with methylation-sensitive restiction enzymes leads to specific digestion products, and these specific digestion products are subjected to amplification by PCR, using a set of primers comprising at least one primer comprising a polynucleotide sequence hybridising selectively with a) a polynucleotide derived from an oil palm plant having a nucleotide sequence that is at least 70% identical to the nucleotide sequence of SEQ ID NO: 1; b) a fragment of polynucleotide a) comprising at least 10 contiguous nucleotides; c) a polynucleotide complementary to polynucleotide a) or b) derived from an oil palm plant. Such a PCR method allows to amplify and detect the biological marker in a rapid screening procedure and at high throughput level.

Alternatively, the degree of methylation in the genomic area represented by SEQ ID NO: 1 or a sequence having at least 70% sequence identity thereto may be determined by chemical modification with bisulfite or affinity-based isolation of methylated DNA. In bisulfite based methods DNA is treated with bisulfite to determine its pattern of methylation. Treatment of DNA with bisulfite convents cytosine to uracil, but leaves 5-methylcytosine unaffected. Bisulfite treatment thus introduces specific changes in the DNA sequence which depend on the methylation status of individual cytosine residues. Various methods may be applied to retrieve information about this methylation status.

Methods applicable for determination of the methylation state after bisulfite treatment comprise non-methylation-specific PCR based methods (e.g. direct sequencing, pyrosequencing, methylation-sensitive single-strand conformation analysis (MS-SSCA), high resolution melting analysis (HRM), methylation-sensitive single-nucleotide primer extension (MS-SnuPE) or base-specific cleavage/MALDI-TOF) or methylation-specific PCR (e.g. by use of methylation specific primers that hybridise to bisulfite converted DNA).

In a particular embodiment of the invention the amplification is performed by contacting the digestion products with a set of primers allowing for amplification of the biomarker. The specific digestion products are preferably ligated with adapters prior to amplification, the adapters being nucleotide sequences.

In a preferred PCR method, genomic DNA of oil palm plants is subjected to methylation sensitive digestion. After digestion the digestion mixture may be ligated with adapters. The thus obtained DNA fragments are subjected to a PCR method using a set of specific primers. Such primers are designed to amplify a DNA product of a specific length depending on whether the site in the genomic DNA corresponding to that product was methylated or not. In addition, such primers are designed to amplify a DNA product of a specific length, in case the site in the genomic DNA corresponding to that product was not methylated. The size of the DNA product in case the product is derived from a site in the genomic DNA that was not methylated differs from that of the DNA product in case the product is derived from a site in the genomic DNA that was methylated. In such a PCR method the analysis is performed by contacting the biological sample with a set of primers comprising at least one primer that is complementary to the genomic area represented by SEQ ID NO: 1 or a sequence having at least 70% sequence identity thereto.

In a preferred embodiment such PCR method may be a multiplex PCR method comprising the utilization of primers in a PCR method to allow amplification of a biological marker of the invention.

Preferably, the set of primers used in the amplification procedure comprises at least one primer hybridising selectively with a) a polynucleotide derived from an oil palm plant having a nucleotide sequence that is at least 70% identical to the nucleotide sequence of SEQ ID NO: 1; b) a fragment of polynucleotide a) comprising at least 10 contiguous nucleotides; c) a polynucleotide complementary to polynucleotide a) or b) derived from an oil palm plant, as well as at least one primer that is able to hybridize with the nucleotide sequence of at least one adapter. For instance, amplification of the ligated digestion products may be performed by contacting the DNA material with a set of primers comprising at least the following components: SEQ ID NO: 2, SEQ ID NO: 3, SEQ ID NO: 4, SEQ ID NO: 5 and SEQ ID NO: 6. In such a multiplex PCR method the analysis is performed by contacting the ligated digestion products with a set of primers comprising at least the oligonucleotides set forth in SEQ ID NO: 2, SEQ ID NO: 3, SEQ ID NO: 4, SEQ ID NO: 5 and SEQ ID NO: 6. When desired, primers may be labeled to facilitate detection of PCR products. Such labels include but are not limited to radioactive labels, such as ³²P and ³³P, fluorescent labels, enzymatic labels, biotin etc. Any suitable PCR amplification program, any suitable PCR device, any suitable PCR buffer, any suitable DNA polymerase, any suitable buffer combination and any way of detecting PCR products may be used as will be understood by the person skilled in the art.

One aspect of the invention comprises that amplification products may be detected via various methods such as electrophoresis, hybridization, amplification, sequencing, ligase chain reaction, chromatography, mass spectrometry and combinations thereof. Electrophoresis may be, for instance agarose based electrophoresis or polyacrylamide based electrophoresis. PCR products may be detected by any suitable method, depending on the labeling of the PCR products, as will be understood by the person skilled in the art.

### Examples

### Plant material

Young leaves from tissue cultured palms (ramets) were sampled from MPOB stations across Malaysia and were also kindly provided by FELDA (Federal Land Development Authority, Malaysia), Agricultural Services Sdn. Bhd., Applied Agricultural Resources Sdn. Bhd., and United Plantations Bhd. The ramets that were selected comprised of normal palm and mantled (abnormal) palm from the same clone (i.e., derived from the same mother palm (ortet)). Leaf samples from the ortet were also obtained if they still existed in the field as some had been felled due to old age or had been destroyed by pests or diseases.

Leaves were also sampled from ramets at nursery stage (6-month old plantlets) in MPOB. Additional nursery stage samples were obtained from FELDA Agricultural Research Sdn. Bhd. which had been previously sampled and stored in the freezer. The sampled ramets were subsequently field-planted and sampling of the leaves were sampled again when the ramets were around 3-4 years of age.

### DNA extraction

Genomic DNA was extracted based on Dellaporta et al. (1983).

### Enzymes and buffers

Enzymes and their corresponding buffers such as restriction buffers, ligation buffers, PCR buffers, T4 forward buffers were purchased and used in accordance with the manufacturer's instructions.

### Example 1: A hypomethylated area in the genome of oil palm plants correlates with the mantled phenotype

### Restriction enzyme digestion and ligation of adapters

The genomic DNA from the clonal sets of normal and abnormal ramets from various laboratories were digested with methylation sensitive isoschizomer enzymes MspI and HpaII. These enzymes are isoschizomers and are sensitive to cytosine methylation. Both do not cut CCGG sites when the external C is methylated but, when the internal C is methylated, MspI is able to cut while HpaII can not (McClelland et al., 1994). The digestion mix of 400 ng DNA, 1X restriction buffer and 10 U of the respective enzyme (New England Biolabs) was incubated at 37°C overnight. Ligation of double stranded adapters, designated GC adapters, was conducted by adding the following mix to the digestion mix: 50 pmoles GC adapters, 1X ligation buffer and 2.5 U T4 ligase (Invitrogen). (NB: GC adapters are generated by combining oligonucleotides according to the sequences SEQ ID NO: 7 (Adapt-short-NH2) and SEQ ID NO: 8 (Adapt-long) in equimolar amounts). The ligation mix was incubated at room temperature overnight.

The degenerate MAD5R primer corresponding to the sequence of SEQ ID NO: 9 was designed based on the conserved MADS-box region from other plant species.

### PCR

Two PCR amplification programmes were consecutively used. The first PCR involves a selective pre-amplification of MADS-box polynucleotide sequences with the primer MAD5R, in a 20 µl PCR mix containing 1X PCR buffer, 0.05 mM dNTPs, 1 pmol MAD5R primer, 2 µl ligation mix from the previous step and 0.015 U HotStar Taq polymerase (Qiagen). The PCR programme used was 95°C for 15 minutes; 30 cycles of 95°C, 30 seconds; 60°C, 1 minute 40 seconds; 72°C, 2 minutes; and a final extension at 72°C, 2 minutes.

The second PCR involved a 40 µl total mix of 1X PCR buffer, 0.25 mM dNTPs, 10 pmol Adapt primer (corresponding to the sequence set forth in SEQ ID NO: 10), 20 pmol MAD5R and 0.0075 U HotStar Taq polymerase and the 20 µl of PCR mix from the first PCR cycle above. The entire mixture was subjected to a PCR programme of 95°C, 15 minutes; 30 cycles of 95°C, 30 seconds; 60°C, 1 minutes 40 seconds; 72°C, 2 minutes; and a final extension at 72°C, 3 minutes. The PCR products were then checked by electrophoresis on a 1.5% agarose in 0.5X TBE buffer.

### End-labelling of primer and PCR product labelling

The primer MAD5R was end-labelled with γ[³³p]-dATP by adding 1X T4 forward buffer, 1 pmol MAD5R, 0.1 U T4 kinase and 0.1 µl γ[³³P]-dATP, incubated at 37°C for at least 3 hours. Subsequently, the PCR products were radioactively labeled by combining 1X PCR buffer, 0.2 mM dNTPs, 0.5 µl end-labeled MAD5R primer, 5 µl PCR product and 0.0075 U HotStar Taq polymerase into a 20 µl reaction mix. The PCR programme used was 95°C, 15 minutes; 10 cycles of 95°C, 30 seconds, 60°C, 1 minute 40 seconds, 72°C, 2 minutes; and a final extension of 72°C, 3 minutes.

### PAGE electrophoresis

The radioactively labeled PCR products were first denatured in a boiling water bath for 5 minutes and immediately cooled on ice. The PCR products (4-5 µl) were then electrophoresed on a 6% polyacrylamide gel at 110W for approximately 2.5 hours. The gel was then vacuum dried at 70°C for approximately 1.5 hours and exposed to X-OMAT film (Kodak) for approximately 5-7 days.

### Result

Following this approach, using MspI- and HpaII-digested DNA from 2 clonal sets, a polymorphic band of 238 bps in size that was amplified from HpaII-digested DNA appeared to associate with mantled and reverted mildly mantled ramets. This suggests that a particular site in the genome is demethylated in the mantled and reverted mantled ramets, but methylated in the ortet and normal ramets, so that the sequence would be converted in the ortet and normal ramets only and not be available for amplification of the band.

### Example 2: Sequence isolation and characterization

### cDNA Isolation

The RACE kit (Clontech, USA) was used according to the manufacturer's instructions.

The 238 bps band was sequenced and has the sequence of SEQ ID NO: 11. Although the PCR assay was intended to amplify MADS-box related polynucleotides, the inventors found to their surprise that the sequence of SEQ ID NO: 11 did not show relation or significant homology to any sequence, whether a gene or an intergenic region, of the MADS-box of *Elaeis guineensis.* Therefore, for further investigation isolation of the surrounding region of the 238 bps region was carried out by genome walking. A genomic region of 4882 bps of *Elaeis guineensis* was sequenced by genome walking and appeared to have the nucleotide sequence as set forth in SEQ ID NO: 1. The region did not produce any significant hits in Genbank. In addition, no open reading frames of sufficient length were found. The isolated polynucleotide of the invention is therefore likely to be derived from an intergenic region in the oil palm's genome.

A further analysis of whether these regions are expressed was done using RACE (rapid amplification of cDNA ends) using primers designed on the obtained genomic regions. No amplification products were obtained using several set of primers, suggesting that the fragment is not part of a gene that is expressed in the tissue used, i.e. a 6.5 cm abnormal female inflorescence.

Three CCGG restriction sites were located in the 4882 bps genomic region. There were various CG and CNG sequences in the 4882 bps isolated region which are possible cytosine methylation sites.

### Example 3: Detection of demethylation to predict the mantled phenotype

A PCR assay was developed to allow rapid screening at high throughput level. The assay involved HpaII digestion followed by ligation with adapters. Subsequently, a multiplex PCR was performed with 5 primers. These were the primers according to sequences SEQ ID NO: 4 (MM78-1 primer), SEQ ID NO: 5 (MM78-2 primer) and SEQ ID NO: 6 (MM78-3 primer), which were designed to hybridize specifically to a polynucleotide sequence contained in the sequence of SEQ ID NO:1. The primers according to sequences SEQ ID NO: 2 (CP1 primer) and SEQ ID NO: 3 (CP2 primer) were designed to hybridize specifically to polynucleotide sequences in the adapter sequences. The procedure was carried out as follows. Genomic DNA (400 ng) was added to a digestion/ligation mixture containing 1X RL+ (restriction/ligation) buffer, 0.8 µM adapter, 1 mM ATP, 5 U HpaII, 1 U T4 DNA ligase and the mixture was incubated at 37°C overnight. The digestion/ligation was terminated by heating at 65°C for 15 minutes. The PCR mix comprised of 10 µl of a 1X diluted digested/ligated DNA, 1X PCR buffer (Qiagen), 0.8 mM dNTP, 0.2 uM MM78-1 primer, 0.2 uM MM78-2 primer, 0.3 uM MM78-3 primer, 25 nM CP1 primer, 25 nM CP2 primer and 0.3 U HotStar Taq polymerase. PCR was conducted at 95°C for 15 minutes, followed by 35 cycles of 95°C for 30 seconds, 65°C for 1 minute, 72°C for 3 minutes and a final extension of 72°C for 3 minutes. The PCR products were then electrophoresed on 2% agarose gel in 1X TBE buffer.

This PCR assay was developed for rapid screening of clonal planting materials for detection of the methylation status of a genomic region of *Elaeis guineensis.* The PCR assay is sensitive, as a low amount of demethylated sites, which produce a small number of fragments, may be amplified up to visible amounts by the amplification reaction.

A product migrating to a size of approximately 650 bps on an electrophoresis gel appears to be generated when the CCGG sequence comprised in positions 2710-2713 (MM78 site) of the sequence of SEQ ID NO:1 is methylated. The sequence of this PCR product was determined and is represented by SEQ ID NO: 25.

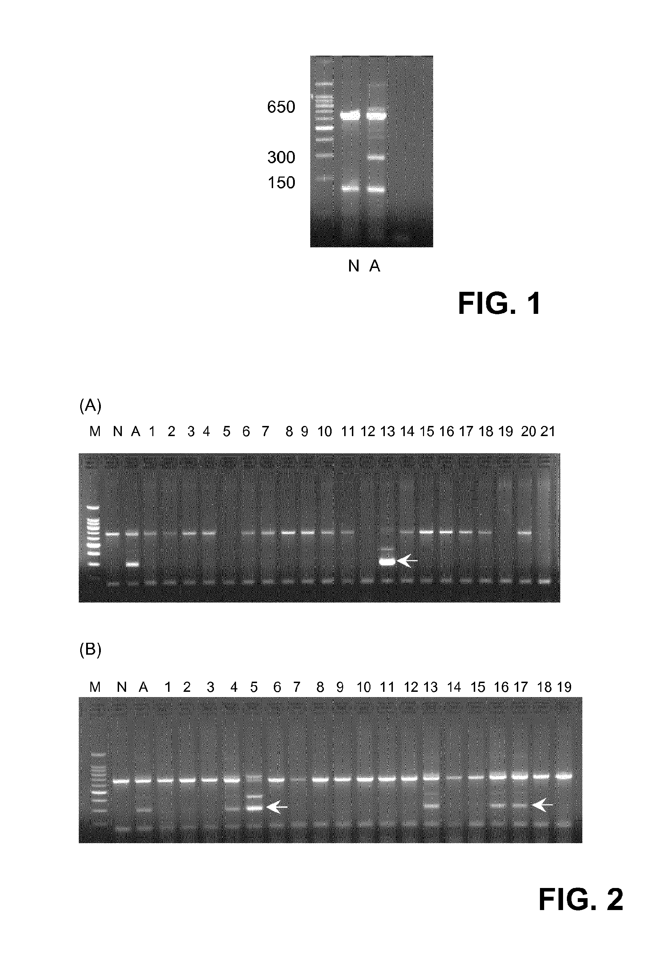

When the site is unmethylated, HpaII will be able to cut at the MM78 position and a shorter product of about 300 bps is generated (Fig.1). In this particular example, the 300 bps product is a biomarker for a mantled phenotype. The sequence of this product was determined and is represented by SEQ ID NO: 26.

The assay was designed to also verify completion of restriction digestion and ligation, which leads to the amplification of a PCR product migrating during electrophoresis to a size of approximately 150 bps, regardless of the methylation status of the restriction site. The sequence of this product was determined and is represented by SEQ ID NO:27.

Thus, the PCR products from this assay are:
● a ∼650 bps product in samples in which the MM78 site is methylated with the sequence of SEQ ID NO: 25,
● a ∼300 bps product in samples in which the MM78 site is demethylated (expected in mantled samples) with the sequence of SEQ ID NO: 26 and,
● a ∼150 bps product that is always amplified with the sequence of SEQ ID NO: 27, which confirms successful digestion and ligation reactions.

Alternatively or in addition, the complementary strand of SEQ ID NO: 1 may be analyzed. In case the HpaII site is unmethylated a product of ∼418 bps is amplified by primers MM78-2 (SEQ ID NO:5) and MM 78-4 (SEQ ID NO: 28). This PCR product was sequenced and its nucleotide sequence is represented by SEQ ID NO: 29.

### Example 4: Correlation of PCR results to mantling

The MM78-PCR assay described in example 3 was used for high-throughput PCR screening of adult ramets of 2 clones and nursery stage ramets. As the MM78 marker was present in several normal ramets from the earlier results, a larger sample set was collected from 2 field-planted clones. MM78-PCR screening of a total of 40 randomly selected field-planted ramets consisting of several mantled palms is shown in (Fig.2). Correlation of the PCR results to the phenotype was conducted, i.e. presence of the MM78 marker to the mantled phenotype and absence of the marker to the normal phenotype. The overall correlation of the PCR results to the field mantling census of the adult palms was tabulated to be about 80%.

The MM78-PCR 300 bps band predicted mantling perfectly in clone P164 ramets (2 mantled out of 21 ramets were both identified as such) but incompletely in clone P149 ramets (only 5 out of 9 mantled ramets showed the 300 bps band)(Table 2). Nevertheless, it still is clear from the occurrence of the 300 bps band that the mantling rate in the set of P149 ramets is higher than in the P164 set. In this set of samples the field mantling rate only refers to mildly mantled phenotype as there were no severely mantled palms observed in these samples prior to and at time of sampling.

**Table 2. Correlation of mantling prediction from MM78-PCR results to field results. Sample size per clone: 21 (P164) and 19 (P149).**

| **Clones** | **Mantling (mild)^{#} rate - field** | **Mantling rate predicted - lab** |
|---|---|---|
| P164 | 2 (4.7%) | 2 (4.7%) |
| P149 | 9 (47%) | 5 (26%) |

| | | |
|---|---|---|
| # mantling rate (field) refers to mild mantling as observed from field census. | | |

### Example 5: Prediction of abnormality correlates at nursery stage to field abnormality rate in several clones

A set of samples consisted of nursery stage ramets and was obtained from FELDA. MM78-PCR screening was performed on samples of 11-34 palms each from four FELDA clones at nursery stage. After they were field-planted their flower morphology was assessed at adult stage after the flowering cycle initiated in the palms. MM78-PCR screening was repeated on these field palms. The overall results suggested correlations of the MM78-predicted abnormality rate to the field abnormality rate of two clones (clone 1 and clone 3) out of the four clones tested (Table 3). However, the abnormality prediction calculated from the MM78-PCR results on the nursery samples did not correlate to the field abnormality rate in all the 4 clones. Therefore, the biological marker of the invention may not be used to predict in an early stage of development a mantled phenotype in oil palm plants of a later age in all clones.

**Table 3. Scoring and correlation of predicted abnormality rates from FELDA's nursery palms and adult palms to field abnormality rates.**

| | **Nursery** | | **Adult/Field** | | **Field data** |
|---|---|---|---|---|---|
| **Clon e No.** | **No. of sampled palms** | **%Abn predictio n¹** | **No. of sampled palms** | **%Abn predictio n¹** | **Mantling % of trial** |
| 1 | 33 | 9 | 40 | **2.5** | **2** |
| 2 | 11 | 55 | 14 | 14.3 | 0 |
| 3 | 28 | 29 | 30 | **6.7** | **6** |
| 4 | 34 | 82 | 29 | 13.8 | 0 |

| | | | | | |
|---|---|---|---|---|---|
| ¹ Abnormality (Abn) prediction based on the presence of the 300 bps MM78-PCR product. | | | | | |

Clones 1 and 3 can be used for micropropagation in culture and after a period of growth, samples can be taken and analysed for hypomethylation as described in example 3. Young plants in which hypomethylation is detected may be removed from the culture. This way the percentage of plants that will eventually develop the mantled phenotype in the field will be minimized.

### Example 6

In an additional experiment samples were taken of a number of flowering oil palm plants of various clonal subsets and analysed as described in example 4. The results for hypomethylation were compared with field data and are shown in table 4. The suitability of the particular clones for association of the marker with the mantled phenotype is indicated with + or -. Table 4 shows that a broad set of clones can be applied to predict the mantled phenotype.

**Table 4: Fruiting ramets of several clones tested with MM78-PCR**

| Clone | Prediction from MM78-PCR results | Number of ramets (% of total ramets) | Field mantling census | Number of ramets (% of total ramets) | suitability |
|---|---|---|---|---|---|
| P10 (n=11) | Predicted normal | 8 (73%) | Field normal | 7 (64%) | + |
| | Predicted abnormal | 3 (27%) | Field abnormal | 4 (36%) | |
| P15 (n=7) | Predicted normal | 5 (71%) | Field normal | 3 (43%) | - |
| | Predicted abnormal | 2 (29%) | Field abnormal | 4 (57%) | |
| P149 (n=23) | Predicted normal | 16 (70%) | Field normal | 11 (48%) | - |
| | Predicted abnormal | 7 (30%) | Field abnormal | 12 (52%) | |
| P164 (n=25) | Predicted normal | 21 (84%) | Field normal | 22 (88%) | + |
| | Predicted abnormal | 4 (16%) | Field abnormal | 3 (12%) | |
| P38 (n=9) | Predicted normal | 2 (22%) | Field normal | 4 (44%) | + |
| | Predicted abnormal | 7 (78%) | Field abnormal | 5 (56%) | |
| P75 (n=7) | Predicted normal | 3 (43%) | Field normal | 4 (57%) | + |
| | Predicted abnormal | 4 (57%) | Field abnormal | 3 (43%) | |
| P273 (n=20) | Predicted normal | 20 (100%) | Field normal | 8 (40%) | - |
| | Predicted abnormal | 0 (0%) | Field abnormal | 12 (60%) | |
| P291 (=11) | Predicted normal | 11 (100%) | Field normal | 4 (36%) | - |
| | Predicted abnormal | 0 (0%) | Field abnormal | 7 (64%) | |
| P297 (n=53) | Predicted normal | 9 (17%) | Field normal | 5 (9%) | + |
| | Predicted abnormal | 44 (83%) | Field abnormal | 48 (91%) | |
| Q38 (n=38) | Predicted normal | 22 (58%) | Field normal | 20 (53%) | + |
| | Predicted abnormal | 16 (42%) | Field abnormal | 18 (47%) | |
| 02/516-557 (n=32) | Predicted normal | 29 (91%) | Field normal | 31 (97%) | + |
| | Predicted abnormal | 3 (9%) | Field abnormal | 1 (3%) | |
| 08/415-449 (n=22) | Predicted normal | 15 (68%) | Field normal | 10 (45%) | - |
| | Predicted abnormal | 7 (32%) | Field abnormal | 12 (55%) | |

### Example 7: Full correlation of hypomethylation with the mantling phenotype in grown plants

Using the approach described in example 3 full correlation in grown palms of the hypomethylation in the polynucleotide represented by sequence SEQ ID NO: 1 to mantling was observed in clonal material from a FC166 clone (FELDA), consisting 18 palms of normal, revertant and mantled phenotypes.

### Example 8: Correlation of hypomethylation with the mantling phenotype in a broad set of clones

Testing 50 samples of tissue of grown palms in total, hypomethylation in the polynucleotide represented by sequence SEQ ID NO: 1 was associated with the mantled palms and/or normal methylation with the normal palms in 10 out of 12 clones tested by using the approach described in example 3. Thus, the biological marker of the invention is applicable in ∼83% of the clones.

### Example 9: Predicting the mantled phenotype using bisulfite sequencing

Approximately 1 µg of genomic DNA was digested with AluI (or other selected enzymes of which the restriction site is not present in the region of interest) in a volume of 21 µl TE overnight. Cold mineral oil (300 µl) was dispensed into 2 ml tubes and left on ice for 30 minutes. Freshly prepared 2M NaOH (4 µl) was added to the digestion mix and incubated for 15 minutes at 50°C. An addition of 50 µl 2% low melting point agarose solution was added to the 25 µl mix. To form DNA beads, 10 µl of this mixture was immediately pipetted into the middle of the mineral oil layer and left on ice for 30 minutes. Subsequently, 500 µl bisulfite solution (5M sodium metabisulfite, 100 mg/ml hydroquinone, pH 5) was gently added to the DNA bead in the mineral oil. The tubes were then incubated at 50°C for 4 hours. Following that, the oil and solution were removed and the DNA bead was washed with 1 ml TE (pH8) for 15 minutes with gentle inversion. This wash was repeated twice. Desulfonation was carried out in 500 µl 0.2M NaOH twice for 15 minutes each time. Treatment was then stopped in 1 ml TE for 10 minutes and the bead can be stored in 1 ml TE overnight at 4°C. Before PCR, the bead was rinsed twice with 1 ml distilled water for 15 minutes each time. The bead was then added into the PCR reaction mix (2.5 µl 10X PCR buffer, 0.5 µl 5mM dNTP, 0.5 µl each primer (10 pmol/µl), 10.92 µl water, 0.08 µl HotStar Taq polymerase. The PCR program used was 95°C, 5 minutes; 50 cycles of 94°C, 30 seconds, 50°C, 30 seconds, 72°C, 45 seconds and a final extension of 72°C, 3 minutes. PCR with nested primers were then carried out with 2 µl of the primary PCR product. Electrophoresis of 10 µl PCR products were conducted in a 2% agarose gel (in 0.5X TBE buffer). Detection of the specific PCR product on the gel woµld lead to sequencing of the PCR product using the same primers used for the PCR of the bisµlfite treated DNA.

A second critical step in the bisulfite sequencing procedure is the primer design. The primers need to bind specifically to converted DNA (in which all Cs have been changed into Ts), but they should not discriminate between unmethylated and methylated DNA. This means that the primers need to anneal at regions devoid of CHG-triplets (where H is A or C or T) and CG-dinucleotides. Consequently, the primer sequence will generally have a lower binding strength, as it is AT-rich. The software used in the primer design for bisulfite sequencing is freely available at http://bisearch.enzim.hu. However, this software was designed for CG methylation studies in human and mammalian DNA and thus the possible methylation (and subsequent conversion of methylated Cs into Ts) in CHG triplets is not taken into account. Thus, the software was used to assign potential primers, but each of the potential primer sequences was checked for the absence of CHG sites. When double-stranded DNA is treated with bisulfite, the C-residues in both strands are converted and thus are no longer complementary. Hence, the primers were designed to either anneal to the converted upper strand or to the converted lower strand.

### Results:

For bisulfite sequencing, the genomic DNA was first digested with AluI, producing several restriction fragments in the MM78 locus and its surrounding region. After bisulfite treatment, the fragments were amplified with primers and nested primers that were designed on the upper strand, and would recognize as much as possible only bisulfite-treated converted DNA. The primers were not designed based on the lower strand. The sequences of the primers are represented by SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 and SEQ ID NO: 24. The expected product size was generated from the PCR and sequencing results verified that the CCGG site (*Hpa*II restriction site) was differentially methylated, as found in the MSMADS profiling in as described in example 3. Bisulfite sequencing therefore can be applied to determine the methylation state of the polynucleotide of the invention.

### References

Adam H, Jouannic S, Escoute J, Duval Y, Verdeil JL, Tregear JW (2005) Reproductive developmental complexity in the African oil palm (Elaeis guineensis, Arecaceae). American J. Botany 92: 1836-1852.
Auyong SMA, Sharifa, SA, Faridah, QZ, Raha, AR, Ong-Abdullah, M (2005) MADS-box directed profiling for the detection of oil palm variations. Proceedings of the PIPOC 2005 International Palm Oil Congress (Agriculture, Biotechnology and Sustainability)
Adam H, Jouannic S, Orieux Y, Morcillo F, Richaud F, Duval Y, Tregear JW (2007) Functional characterization of MADS box genes involved in the determination of oil palm flower structure. J. Exp. Bot. 58: 1245-1259.
Arnholdt-Schmitt B, Holzapfel B, Schillinger A, Neumann KH (1991) Variable methylation and differential replication of genomic DNA in cultured carrot root explants during growth induction as influenced by hormonal treatments. Theor. Appl. Genet. 82: 283-288.
Breure K (2003) The search for yield in oil palm: Basic principles. In: Fairhurst TH, Hardter R eds) Oil Palm: Management for Large and Sustainable Yields. PPI, PPIC and IPI, Singapore, Canada, Switzerland, pp 59-91.
Coen ES, Meyerowitz EM (1991) The war of the whorls: genetic interactions controlling flower development. Nature 353: 31-37.
Cohen Y, Korchinsky R, Tripler E (2004) Flower abnormalities cause abnormal fruit setting in tissue culture propagated date palm (Phoenix dactylifera L.). J. Hort. Sci. Biotech. 79: 1007-1013.
Corley RHV, Law IH (1997) The future for oil palm clones. In: Proceedings of the International Planters Conference. pp 279-289.
Corley RHV, Lee CH, Law IH, Wong CY (1986) Abnormal flower development in oil palm clones. Planter 62: 233-240.
Corley RHV, Wong CY, Wooi KC (1982) Early results from the first oil palm clone trials. In: Pushparajah E, Chew PS eds) The oil palm in agriculture in the eighties. The Incorporated Society of Planters, Kuala Lumpur, pp 173-196.
Dellaporta SL, Wood J, Hicks JB (1983) A plant DNA minipreparation: version II. Plant Mol. Biol. Rep. 1: 19-21. Duval Y, Amblard P, Rival A, Konan E, Gogor A, Durrand-Gasselin T (1997) Progress in oil palm tissue culture and clonal performance in Indonesia and the Cote d'Ivoire. In: Proceedings of the International Planters Conference. pp 291-307.
Eeuwens CJ, Lord S, Donough CR, Rao V, Vallejo G, Nelson S (2002) Effects of tissue culture conditions during embryoid multiplication on the incidence of "mantled" flowering in clonally propagated oil palm. Plant Cell, Tissue and Organ Culture 70: 311-323.
Finnegan EJ, Genger RK, Peacock WJ, Dennis ES (1998) DNA methylation in plants. Annu. Rev. Plant Physiol. Plant Mol. Biol. 49: 223-247.
Hardon JJ, Corley RHV, Lee CH (1987) Breeding and selecting the oil palm. In: Abbot AJ, Atkin RK eds) Improving vegetatively propagated crop. Academic Press, London, pp 63-81.
Hardter R, Fairhurst T (2003) Introduction. In: Fairhurst TH, Hardter R eds) Oil Palm: Management for Large and Sustainable Yields. PPI, PPIC and IPI, Singapore, Canada, Switzerland, pp 1-12.
Henikoff & Henikoff, 1992, PNAS 89, 915-919
Jaligot E, Adler S, Debladis E, Beule T, Richaud F, Ilbert P, Finnegan EJ, Rival A (2011) Epigenetic imbalance and the floral developmental abnormality of the in vitro-regenerated oil palm Elaeis guineensis. Annals of Bot.: doi:10.1093/aob/mcq266.
Jaligot E, Rival A, Beule T, Dussert S, Verdeil JL (2000) Somaclonal variation in oil palm (Elaeis guineensis Jacq.): the DNA methylation hypothesis. Plant Cell Reports 19: 684-690.
Jones LH (1974) Propagation of clonal palms by tissue culture. . Oil Palm Newsletter 17: 1-8.
Jullien PE, Berger F (2010) DNA methylation reprogramming during plant sexual reproduction? Trends Genet 26: 394-399. Kaeppler SM, Kaeppler HF, Rhee Y (2000) Epigenetic aspects of somaclonal variation in plants. Plant Mol. Biol. 43: 179 - 188.
Kaeppler SM, Phillips RL (1993) DNA methylation and tissue culture-induced variation in plants. In Vitro Cell Dev. Biol. 29: 125-130.
Kang HG, Jeon JS, Lee S, An G (1998) Identification of class B and class C floral organ identity genes from rice plants. Plant Mol. Biol. 38: 1021-1029.
Kubis SE, Castilho AMMF, Vershinin AV, S H-HJ (2003) Retroelements, transposons and methylation status in the genome of oil palm (Elaeis guineensis) and the relationship to somaclonal variation. Plant Mol. Biol. 52: 69-79.
LoSchiavo F, Pitto L, Giuliano G, Torti G, Nuti-Ronchi V, Marazziti D, Vergara r, Orselli S, Terzi M (1989) DNA methylation of embryogenic carrot cell cultures and its variations as caused by mutation, differentiation, hormones and hypomethylating drugs. Theor. Appl. Genet. 77: 325-331. Luff B, Pawlowski L, Bender J (1999) An inverted repeat triggers cytosine methylation of identical sequences in Arabidopsis. Mol. Cell. 3: 505-511.
Maheran AB, Abu Zarin O (1999) FELDA's experience in planting on inland soils. In: Proceedings of the 1999 PORIM International Palm Oil Congress. Kuala Lumpur, pp 330-343. Maheran AB, Aw KT, Abu Zarin O, Chin CW (1993) Vegetative propagation of oil palm (Elaeis guinensis) from laboratory to field - FELDA's experience. In: Jalani S, Ariffin D, Rajanaidu N, Tayeb MD, Paranjothy K, Basri MW, Henson IE, Chang KC (eds) Proceedings of the 1993 PORIM International Palm Oil Congress Palm Oil Research Institute of Malaysia, Kuala Lumpur, pp 99-113.
Matzke M, Matzke AJM (1993) Genomic Imprinting in Plants: Parental Effects and Trans-Inactivation Phenomena. Annual Review of Plant Physiology and Plant Molecular Biology 44: 53-76.
Mohd Basri W, Siti NAA, Henson IE (2004) Oil palm - achievements and potential. In: Fischer T, Turner N, Angus J, McIntyre L, Robertson M, Borrell A, Lloyd D (eds) Proceedings for the 4th International Crop Science Congress: New directions for a diverse planet. Regional Institute Ltd, Australia.
Purugganan MD, Rounsley SD, Schmidt RJ, Yanofsky MF (1995) Molecular evolution of flower development: Diversification of the plant MADS-box regulatory gene family. Genetics 140: 345-356.
Rabéchault H, Martin J-P (1976) Multiplication végétative du palmier á huile (elaeis guineensis Jacq.) á l'aide de cultures de tissues foliares. C.R.Acad. Sci. Paris. 283: 1735-1737. Rajanaidu N, Kushairi A, Chan KW, Mohd Din A (2007) Current status of oil planting material in the world and future challenges. In: Proceedings of the PIPOC 2007 International Palm Oil Congress. Malaysian Palm Oil Board, Kuala Lumpur, pp 503-520.
Sablowski R (2010) Genes and functions controlled by floral organ identity genes. Seminars in Cell Dev. Biol. 21: 94-99. Simon S, Hendry T, Chang SW, Kiaw CW (1998) Early yield performance of clonal oil palm (Elaeis guineensis Jacq.) plantings in PBB Oil Palm Bhd., Sabah - a case study. The Planter 74: 257-269.
Smulders M,JM de Klerk GJ (2011) Epigenetics in plant tissue culture. Plant Growth Regul 63: 137-146.
Smulders MJM, Rus-Kortekaas W, Vosman B (1995) Tissue culture-induced DNA methylation polymorphisms in repetitive DNA of tomato calli and regenerated plants. Theoretical and Applied Genetics 91: 1257 - 1264.
Soh AC, Wong G, Tan CC, Chew PS, Hor TY, Chong SP, Gopal K (2001) Recent advances towards commercial production of elite oil palm clones.. In: Proceedings of the 2001 PIPOC. Kuala Lumpur, pp 33-44.
Syed Alwee S, van der Linden CG, van der Schoot J, de Folter S, Angenent G, Cheah S-C, Smulders MJM (2006) Characterization of oil palm MADS box genes in relation to the mantled flower abnormality. Plant Cell Tissue Organ Culture 85: 331-344.
Tandre K, Svenson M, Svensson ME, Engstrom P (1998) Conservation of gene structure and activity in the regulation of reproductive organ development of conifers and angiosperms. Plant J. 15: 615-623.
Theissen G (2001) Development of floral organ identity: stories from the MADS house. Curr. Opin. Plant Biol. 4: 75-85. Weigel D, Nilsson O (1995) A developmental switch sufficient for flower initiation in diverse plants. Nature 377: 495-500. WO03/040297
Wooi KC (1993) Oil palm tissue culture - current practice and constraints. In: Rao V, Henson IE, Rajanaidu N (eds) Proceedings of the 1993 ISOPB International Symposium on Recent Developments in Oil Palm Tissue Culture and Biotechnology. PORIM, Kuala Lumpur, pp 21-32.
Zahn LM, Leebens-Mack J, De Pamphilis CW, Ma H, Theissen G (2005) To B or not to B a flower: the role of DEFICIENS and GLOBOSA orthologs in the evolution of angiosperms. J. Heredity 96: 225-240.

## Claims

1. An isolated polynucleotide selected from the group consisting of:
a) a polynucleotide derived from an oil palm plant having a nucleotide sequence that is at least 70% identical to the nucleotide sequence of SEQ ID NO: 1;
b) a fragment of polynucleotide a) comprising at least 10 contiguous nucleotides;
c) a polynucleotide complementary to polynucleotide a) or b) derived from an oil palm plant; and
d) a polynucleotide hybridising selectively with polynucleotide a), b) or c).

2. A nucleic acid construct comprising the polynucleotide of claim 1.

3. A use of the polynucleotide of claim 1 or the nucleic acid construct of claim 2 for predicting the mantled phenotype in oil palm plants.

4. The use according to claim 3 for predicting in young oil palm plants the development of a mantled phenotype in a later stage of their life cycle.

5. A method for predicting a mantled phenotype in an oil palm plant, wherein DNA material, comprising the nucleotide sequence of the polynucleotide of claim 1 a), b), or c), is isolated from said plant and the degree of methylation is analysed in a region of said DNA material having said nucleotide sequence.

6. The method according to claim 5, for predicting in young oil palm plants the development of a mantled phenotype in a later stage of their life cycle.

7. A method for minimizing the percentage of oil palm plants with a mantled phenotype in a culture, wherein DNA material, comprising the nucleotide sequence of the polynucleotide of claim 1 a), b), or c), is isolated from oil palm plants and analysed for the degree of methylation in a region of said DNA material having said nucleotide sequence, wherein plants wherein the DNA in said region is hypomethylated are removed from the culture.

8. The method according to claim 7, wherein the oil palm plants from which DNA is isolated are young plants.

9. The method according to claim 8, wherein the young plants are *in vitro* cultures.

10. The method according to claim 8 or 9, comprising the following subsequent steps:
i) isolating DNA material, comprising the nucleotide sequence of the polynucleotide of claim 1 a), b) or c), from a plurality of young plants of a plurality of oil palm clones;
ii) analysing for the degree of methylation in a region of said DNA having said nucleotide sequence,
iii) determining in which clones hypomethylation in said region is indicative for developing the mantled phenotype,
iv) selecting clones in which hypomethylation in said region is indicative for developing the mantled phenotype; and
v) maintaining in the culture the selected clones wherein less than 50% of the plants is hypomethylated in said region and wherein less than 50% of the plants develops a mantled phenotype, while removing the rest of the clones from the culture.

11. The method according to claim 10, wherein in step v) the selected clones wherein less than 5% of the plants is hypomethylated in said region and wherein less than 5% of the plants develops a mantled phenotype are maintained in the culture, while the rest of the clones are removed from the culture.

12. The method according to claim 10 or 11, comprising the additional steps:
vi) repeating steps i) and ii) in young plants derived from the clones selected in step iv); and
vii) removing the plants which are hypomethylated in said region from the culture.

13. The method for predicting a mantled phenotype in an oil palm plant according to claim 5 or 6 or the method for minimizing the percentage of oil palm plants with a mantled phenotype in a culture according to any of the claims 7 to 12, wherein said DNA material is subjected to a procedure selected from the group comprising:
a) chemical modification with bisulfite;
b) affinity-based isolation of methylated DNA;
c) amplification by PCR; and
d) treatment with methylation-sensitive restriction enzymes; or a combination thereof.

14. The method according to claim 13, wherein treatment with methylation-sensitive restriction enzymes leads to specific digestion products, wherein said specific digestion products are subjected to amplification by PCR, using a set of primers comprising at least one primer comprising a polynucleotide sequence according to claim 1 d).

15. The method according to claim 14, wherein said specific digestion products are ligated with adapters prior to amplification, the adapters being nucleotide sequences.

16. The method according to claim 15, wherein the set of primers comprises at least one primer according to claim 1 d), as well as at least one primer that is able to hybridize with the nucleotide sequence of at least one adapter.

17. The method according to claim 16, wherein amplification is performed by contacting said construct with a set of primers comprising at least the following components: SEQ ID NO: 2, SEQ ID NO: 3, SEQ ID NO: 4, SEQ ID NO: 5 and SEQ ID NO: 6.

18. The method according to any of the claims 5-17, comprising a detection method selected from the group consisting of electrophoresis, hybridization, amplification, sequencing, ligase chain reaction, chromatography, mass spectrometry and combinations thereof.


SEQUENCE LISTING

<110> Malaysian Palm Oil Board

<120> A method for predicting a mantled phenotype in oil palm plants and
polynucleotide to be used in such method

<130> EPP177270

<160> 29

<170> BiSSAP 1.2

<210> 1
<211> 4882
<212> DNA
<213> Elaeis guineensis

<220>
<221> source
<222> 1..4882
<223> /mol_type="unassigned DNA"
      /organism="Elaeis guineensis"

<400> 1
gtatagaact cttwgarcgt ttagataaca aaaaatatga ttgttgctgt cggggagtca      60

agctggctca ggctgctcag ctagagtcag gggagagaga gaacaaatga tggccaccaa     120

atgtcaattg agatctccga gttgggtgct agtgtcagct cttcgattga tccctcgagc     180

tagatcctgc aaccatcaaa gataaaaaaa gtcctcatgc tggaggagag gctcctcagt     240

ggtggaccct cccgatacct aaaccagtct cccacaaaag taaatataag atatttgagt     300

ttttagaaag agagttcaac aatggagtca agtgtgaagt atggcagatt agagaatgaa     360

agttcataag gtgcaatgtg gaagattgag ttccaaaatg gagtcagaga aacttagagc     420

tctatggagg agatagattt cagttcgtat ataatcagtc actgagagaa gccatgtgga     480

gtaagagtty tcctagttta ctcagaggat cttggggagt tgggttttat aaatgaaggt     540

ggatatctat cgtctaaggg aatgcatgcc attttgkcta gtgagttgtg gtcatgaaaa     600

catgctttag ctgttagcta gtaatggstc acacacgttt gggatttgtt ccgatkakgg     660

akatcmmyrh aksgamaaaa aagtagggca acgagatggc ttgctgctta tcatctagta     720

gatagaagac tctaaagctc ctcctgttag gaccataaaa gatcaagtgg cagaggtagg     780

atggtcactc tatcatcttt gattgaagat acatgtcaga ctactgctgg atcaccagtg     840

ccacagtgaa tagtttatct tacgatgtat caattgcctc ccccccccac ccgcttccaa     900

gggtaaraaa tctttttggc tcgaataaaa aaagtaraaa gtactcttgc atttattata     960

gcattggtgg ttagggtcaa tgctgaagct ctagattgac cctccaatgc aaatgctggt    1020

gagagtcttc aagatgcagt cttgtcgagg catgctttga cgggcatgca agataaagta    1080

gggagctttg atgtcatatg ccaccggggt tgttccttgg gaggccacga cttgggtaga    1140

tgattggacg tgtgcttgcc ttcttttctc gagagggcat catggtgcac atattttcta 1200

gcatggtgat tggatagctc aacagtgttg atccaaatgg catttcaatg tgtagaggag 1260

tgatgacatg tataaatcaa tcagactatc agccaatttg tttatcatca tcaagccatg 1320

ttatataaat ggcatagaag agacaggcat cacccacctt gcctcctatt tgtagagttg 1380

aagggaggtg ttggggcagc cgtagctgtt gtcttcctct tgcctaagat ttttgatggg 1440

tctacctctt ctcccatcct tccttttttt tagttccttt ckttgttttg cattcatctt 1500

gatggcttct tctatgaaaa aactagtaga atagggatcc actaatctag gatcacttgc 1560

ccaggcttct aagggctcta ccatagaagc acccgaaaat catgaaactc cggatatggt 1620

taagttagtc tcagaggatg atgatctcaa atggatctga aagtagtatg ggattttgaa 1680

aagtagtcct tgaaatgccc agcctctctg gtaaggtcac ttcctctcac ggaaatagga 1740

taatcctata tgaggaggcc ttaagggcca gccttgggct cctaattctc gaggtctttg 1800

tcgaactact gggatggtac cagattkatc ttacccaact catccccaat gtttgacaga 1860

tggtagttga gttcttttgt ttcctcttgc tcttgagggt acctcttcat gtgaagatgt 1920

ttagagcatt catccaggtg aaggcacatt ctcgaagcag aggctggtgg tgcacttctc 1980

ctaggaaagg gatcaccaac ttttcctcyg acccctccat tcatgagtgg aaaagccaat 2040

tcttcttcgc atcctcctca actccatgga gatttctaac tagtgaggat cgtcgatgaa 2100

ctctatgata ttgaagtttc cagagttgtc ctaaacagag gagaagaggg tggatcagtt 2160

gaagcgactc aaggtaacat catactccaa attcttacac aaggaccttc ttaaaaagct 2220

atattttgga ggtctagccc tcagaggtga gtattggtgt ttgtttgttt aacccctaag 2280

ctatcaggcc aaaacatgct ttcttagtgc tcatgcaggt ctagtattct ctcaagaaga 2340

gctagctagt gctagtcctc ccaaggagaa ggaaaaaaag gagaagagag gaagaggatt 2400

gctctatccc cttctcatgg ggttgctatg gtcgaggcta ctgctgcaaa ggctgttgtt 2460

atggaggttg atgctgctga agctaagatt gctgaaacta cagatcagca aagggagagg 2520

tgatcaaaga ggaagcatgc caccccttca tctgaggtca tagaggttat cacaatcgat 2580

gtaactccta tcgaaggcat ttctcctaaa gttataaggg aatatacccc aattctccca 2640

aacattcaac aacctcctac tagtagttcg gtgccagtgg ctgaggctta tacgaaactg 2700

ctaggagaac cggtgccttc cgtcgaaaat gagaggctgc aggcatggtt ttttggagac 2760

tgccttcaag atgctatcaa ggtaaggtct tggcatcgac tttcttttgg tatatcttca 2820

ttctgatctg atggtggtct tcaaggagcc ttggtaatgt gaggcctact tcaataggtt 2880

caatgcttgg atgaagaagc ttgccactcc cgccaccatc tctaggtagc tgaagaggag 2940

cgagaacaaa atagaaaaag ggttgcattc tatcctgatg cagtcaaagg agtagcttac 3000

tatagagaag tctcgagctg acacagtaga ggctgctttt ggggaagcca agacctctct 3060

aaaagaagtt aagaatcttt gaagataaag gttgctcagc cccaagagag tcaagttaag 3120

gctacttgct tgcgggatca gctggcgagg actaaaagga ctgtgcacca agctagagaa 3180

aaggtggcct aggcaagcga ggctcgatct tagttggcag ataaggtcac tactatcaag 3240

tgccatctta tgaaagcaac taccgaagac atggagccta caaacagagc aagacatttt 3300

ataaagagtt gctgaaatca tgctgggagg ccttcttcac tatcttccac tagtgtaaga 3360

agaagatagg gtcataattt ttagagattg atcttaatct tatattagtg ctggcctcat 3420

cttgctcaag cagtggtttg gctaatgcag aaggagatgg aaacatggag ggtgatgcta 3480

agtggcacct ccaattaatt tttctttttc tttttgtaac ttaactgata aaattttgta 3540

acccaagaga agctatcaga aatgaatgaa ttcttttgga gcttcaattt ctttcttctt 3600

tttccttgcr tgcatgacct cattctatga ttttcttgct tggacaaaga tgaagttcat 3660

catggaggag ttgttgggtg gyttgtcaaa ggtgaagagg aggactaccc acactggatt 3720

gagtcgaaga ataggtggat agggttcaat tgtgccctaa catccctggc aagatccttc 3780

agaccgggcc ttcttcaggc acagccaagg atactctttt gattggtgtc gaggccggtg 3840

accaatccaa taggactgtc aagtcaggtc tgctgaaaac ttaaagaggc ctattcgata 3900

caacaccact ttcggatgcc tcaagwgttt ggctcgttaa tgctggaatc tgagtcgagg 3960

cacaaagcca cttgccaagc tggggtcgga agcagctacc ttggatacga tggagattgt 4020

aggccaagat gtcgtggtgg agtctccatc catcatcatc aaagtcttgg gcatggcagc 4080

cgagatgtca taggctgcct gtgtagggac caaaggttaa ccacacaggt gaggattgtg 4140

ataggcttaa ttgaattaag gaggtgattg aggaagaagc aatcatcatg gcgatcgaga 4200

tggtgggaga gactgtgggt cctctagtta tgcagtcaaa gtgtcacctt cctatcttcc 4260

aattgagcct tttcttccca ctgcctctag gcgagctctt cgtcctttca ctcctagatg 4320

gttgaaccaa gaggatgaga tggatatctc ctcggcatga agagtaccta tccaaaataa 4380

ccaatgaaga ggcactgcta agctcatatc acagcatccc aattgtgagt ttttttcctt 4440

tgctcgatgc ttrtttttgc atttttcrtt gactaatcgg ccaactctac aaatggattt 4500

tgctagccaa gcattggagg ctcgactggt atcgcatgat caggtatggg cagtcgagaa 4560

aagyattttt tagatcgagc atcaggaaag gatcattgag catggctaag ccctgatgga 4620

actaggacaa agcaccatca aggaggttcg agagaatctc taaagataac tcaataattt 4680

tcttatcaaa aatgagaagt tgaagaatag cttggaggtt tctcagtggg aggttgaaag 4740

ccgaaagaag aagggtcctt agttgcttga ggcttctcgt ctcccttcct atgaagcaag 4800

agatatctag tcggctcatg tgaggaagtg tgagcacctt tgaccaagac attcagcaga 4860

gaggstagcc ttcatcatga aa 4882

<210> 2
<211> 24
<212> DNA
<213> Artificial Sequence

<220>
<221> source
<222> 1..24
<223> /mol_type="unassigned DNA"
      /note="primer hybridizing to adapter sequence (CP1 primer)"
      /organism="Artificial Sequence"

<400> 2
aaattcccat tggctgacac tgtg                                              24


<210> 3
<211> 29
<212> DNA
<213> Artificial Sequence

<220>
<221> source
<222> 1..29
<223> /mol_type="unassigned DNA"
      /note="Primer hybridizing to adapter sequence (CP2 primer)"
      /organism="Artificial Sequence"

<400> 3
caacccgaaa gtatagatcc cacggctcg                                         29


<210> 4
<211> 23
<212> DNA
<213> Artificial Sequence

<220>
<221> source
<222> 1..23
<223> /mol_type="unassigned DNA"
      /note="primer designed based on SEQ ID NO: 1 (MM78-1 primer)"
      /organism="Artificial Sequence"

<400> 4
gctgcaaagg ctgttgttat gga                                               23


<210> 5
<211> 23
<212> DNA
<213> Artificial Sequence

<220>
<221> source
<222> 1..23
<223> /mol_type="unassigned DNA"
      /note="Primer designed based on SEQ ID NO: 1 (MM78-2 primer)"
      /organism="Artificial Sequence"

<400> 5
cttggggctg agcaaccttt atc                                               23

<210> 6
<211> 29
<212> DNA
<213> Artificial Sequence

<220>
<221> source
<222> 1..29
<223> /mol_type="unassigned DNA"
      /note="Primer designed based on SEQ ID NO: 1 (MM78-3 primer)"
      /organism="Artificial Sequence"

<400> 6
caacccgaaa gtatagatcc cacggttct                                    29


<210> 7
<211> 16
<212> DNA
<213> Artificial Sequence

<220>
<221> source
<222> 1..16
<223> /mol_type="unassigned DNA"
      /note="Primer designed to generate GC adapter (Adapt-short-NH2) "
      /organism="Artificial Sequence"

<400> 7
cgtgggatct atactt                                                  16


<210> 8
<211> 32
<212> DNA
<213> Artificial Sequence

<220>
<221> source
<222> 1..32
<223> /mol_type="unassigned DNA"
      /note="Primer designed to generate GC adapter (Adapt-long)"
      /organism="Artificial Sequence"

<400> 8
actcgattct caacccgaaa gtatagatcc ca                                32


<210> 9
<211> 20
<212> DNA
<213> Artificial Sequence

<220>
<221> source
<222> 1..20
<223> /mol_type="unassigned DNA"
      /note="Degenerate primer designed based on the conserved MADS-box
      region from various plant species (MAD5R primer)"
      /organism="Artificial Sequence"

<400> 9
gttctckatc ckcttsakct                                                    20


<210> 10
<211> 21
<212> DNA
<213> Artificial Sequence

<220>
<221> source
<222> 1..21
<223> /mol_type="unassigned DNA"
      /note="Primer hybridizing to adapter sequence (Adapt primer)"
      /organism="Artificial Sequence"

<400> 10
actcgattct caacccgaaa g                                                  21


<210> 11
<211> 238
<212> DNA
<213> Elaeis guineensis

<220>
<221> source
<222> 1..238
<223> /mol_type="unassigned DNA"
      /note="Sequence of DNA in polymorphic band associating with mantl
      ed phenotype"
      /organism="Elaeis guineensis"

<400> 11
ccggtgcctt ccgtcgaaaa tgagaggctg caggcatggt tttttggaga ctgccttcaa      60

gatgctatca aggtaaggtc ttggcatcga ctttcttttg gtatatcttc attctgatct     120

gatggtggtc ttcaaggagc cttggtaatg tgaggcctac ttcaataggt tcaatgcttg     180

gatgaagaag cttgccactc ccgccaccat ctctaggtag ctgaagagga gcgagaac       238


<210> 12
<211> 24
<212> DNA
<213> Artificial Sequence

<220>
<221> source
<222> 1..24
<223> /mol_type="unassigned DNA"
      /note="Bisulfite sequencing primer (BS78-1F1)"
      /organism="Artificial Sequence"

<400> 12
gttttatatt attaaggttt tttg                                               24


<210> 13
<211> 22
<212> DNA
<213> Artificial Sequence

<220>
<221> source
<222> 1..22
<223> /mol_type="unassigned DNA"
      /note="Bisulfite sequencing primer (BS78-1R1)"
      /organism="Artificial Sequence"

<400> 13
ataccacccc ttcatctraa at                                                22


<210> 14
<211> 22
<212> DNA
<213> Artificial Sequence

<220>
<221> source
<222> 1..22
<223> /mol_type="unassigned DNA"
      /note="Bisulfite sequencing primer designed based on SEQ ID NO: 1
      (BS78-1F2)"
      /organism="Artificial Sequence"

<400> 14
gttaagattt tattttgata gt                                                22


<210> 15
<211> 20
<212> DNA
<213> Artificial Sequence

<220>
<221> source
<222> 1..20
<223> /mol_type="unassigned DNA"
      /note="Bisulfite sequencing primer (BS78-1R2)"
      /organism="Artificial Sequence"

<400> 15
aaaacatttc tcctaaaatt                                                   20


<210> 16
<211> 23
<212> DNA
<213> Artificial Sequence

<220>
<221> source
<222> 1..23
<223> /mol_type="unassigned DNA"
      /note="Bisulfite sequencing primer (BS78-2F1)"
      /organism="Artificial Sequence"

<400> 16
agtttggtaa gtggttttgt gtt                                               23


<210> 17
<211> 23

<212> DNA
<213> Artificial Sequence

<220>
<221> source
<222> 1..23
<223> /mol_type="unassigned DNA"
      /note="Bisulfite sequencing primer (BS78-2R1)"
      /organism="Artificial Sequence"

<400> 17
acctcattct ataattttct tac                                              23


<210> 18
<211> 19
<212> DNA
<213> Artificial Sequence

<220>
<221> source
<222> 1..19
<223> /mol_type="unassigned DNA"
      /note="Bisulfite sequencing primer (BS78-2F2)"
      /organism="Artificial Sequence"

<400> 18
gagttaaatw ttgaggtat                                                   19


<210> 19
<211> 21
<212> DNA
<213> Artificial Sequence

<220>
<221> source
<222> 1..21
<223> /mol_type="unassigned DNA"
      /note="Bisulfite sequencing primer (BS78-2R2)"
      /organism="Artificial Sequence"

<400> 19
aactacccac actyaattaa a                                                21


<210> 20
<211> 20
<212> DNA
<213> Artificial Sequence

<220>
<221> source
<222> 1..20
<223> /mol_type="unassigned DNA"
      /note="Bisulfite sequencing primer (BS78-3F1)"
      /organism="Artificial Sequence"

<400> 20
taagaatttg gagtatgatg                                                  20


<210> 21

<211> 21
<212> DNA
<213> Artificial Sequence

<220>
<221> source
<222> 1..21
<223> /mol_type="unassigned DNA"
      /note="Bisulfite sequencing primer (BS78-3R1)"
      /organism="Artificial Sequence"

<400> 21
   acccctccat tcataaataa a                                              21


<210> 22
<211> 20
<212> DNA
<213> Artificial Sequence

<220>
<221> source
<222> 1..20
<223> /mol_type="unassigned DNA"
      /note="Bisulfite sequencing primer (BS78-3F2)"
      /organism="Artificial Sequence"

<400> 22
atttggagta tgatgttatt                                                 20


<210> 23
<211> 24
<212> DNA
<213> Artificial Sequence

<220>
<221> source
<222> 1..24
<223> /mol_type="unassigned DNA"
      /note="Bisulfite sequencing primer (BS78-4F1)"
      /organism="Artificial Sequence"

<400> 23
tttttattta tgaatggagg ggtt                                            24


<210> 24
<211> 23
<212> DNA
<213> Artificial Sequence

<220>
<221> source
<222> 1..23
<223> /mol_type="unassigned DNA"
      /note="Bisulfite sequencing primer (BS78-4R1)"
      /organism="Artificial Sequence"

<400> 24
ttcctcttac tcttaaaaat acc                                             23

<210> 25
<211> 664
<212> DNA
<213> Artificial Sequence

<220>
<221> source
<222> 1..664
<223> /mol_type="unassigned DNA"
      /note="PCR-product in samples in which MM78 site is methylated"
      /organism="Artificial Sequence"

<400> 25
gctgcaaagg ctgttgttat ggaggttgat gctgctgaag ctaagattgc tgaaactaca      60

gatcagcaaa gggagaggtg atcaaagagg aagcatgcca ccccttcatc tgaggtcata     120

gaggttatca caatcgatgt aactcctatc gaaggcattt ctcctaaagt tataagggaa     180

tataccccaa ttctcccaaa cattcaacaa cctcctacta gtagttcggt gccagtggct     240

gaggcttata cgaaactgct aggagaaccg gtgccttccg tcgaaaatga gaggctgcag     300

gcatggtttt ttggagactg ccttcaagat gctatcaagg taaggtcttg gcatcgactt     360

tcttttggta tatcttcatt ctgatctgat ggtggtcttc aaggagcctt ggtaatgtga     420

ggcctacttc aataggttca atgcttggat gaagaagctt gccactcccg ccaccatctc     480

taggtagctg aagaggagcg agaacaaaat agaaaaaggg ttgcattcta tcctgatgca     540

gtcaaaggag tagcttacta tagagaagtc tcgagctgac acagtagagg ctgcttttgg     600

ggaagccaag acctctctaa aagaagttaa gaatctttga agataaaggt tgctcagccc     660

caag                                                                  664


<210> 26
<211> 292
<212> DNA
<213> Artificial Sequence

<220>
<221> source
<222> 1..292
<223> /mol_type="unassigned DNA"
      /note="PCR product in samples in which MM78 site is demethylated"
      /organism="Artificial Sequence"

<400> 26
gctgcaaagg ctgttgttat ggaggttgat gctgctgaag ctaagattgc tgaaactaca      60

gatcagcaaa gggagaggtg atcaaagagg aagcatgcca ccccttcatc tgaggtcata     120

gaggttatca caatcgatgt aactcctatc gaaggcattt ctcctaaagt tataagggaa     180

tataccccaa ttctcccaaa cattcaacaa cctcctacta gtagttcggt gccagtggct     240

gaggcttata cgaaactgct aggagaaccg tgggatctat actttcgggt tg             292


<210> 27

<211> 149
<212> DNA
<213> Artificial Sequence

<220>
<221> source
<222> 1..149
<223> /mol_type="unassigned DNA"
      /note="PCR product confirming succesful digestion and amplificati
      on"
      /organism="Artificial Sequence"

<400> 27
aaattcccat tggctgacac tgtgcggtca tctttgccgc ccccgacggc caccgagtcg      60

agcgcacgtc cccctccgta ctgattggat gcgccacatc gcgcgctcat ttgtatgccg     120

cgagccgtgg gatctatact ttcgggttg                                       149


<210> 28
<211> 29
<212> DNA
<213> Artificial Sequence

<220>
<221> source
<222> 1..29
<223> /mol_type="unassigned DNA"
      /note="Primer designed based on SEQ ID NO: 1 (MM78-4)"
      /organism="Artificial Sequence"

<400> 28
caacccgaaa gtatagatcc cacggtgcc                                       29


<210> 29
<211> 418
<212> DNA
<213> Artificial Sequence

<220>
<221> source
<222> 1..418
<223> /mol_type="unassigned DNA"
      /note="PCR product when MM78 site is demethylated (analysis of co
      mplementary strand of SEQ ID NO: 1)"
      /organism="Artificial Sequence"

<400> 29
caacccgaaa gtatagatcc cacggtgcct tccgtcgaaa atgagaggct gcaggcatgg      60

ttttttggag actgccttca agatgctatc aaggtaaggt cttggcatcg actttctttt     120

ggtatatctt cattctgatc tgatggtggt cttcaaggag ccttggtaat gtgaggccta     180

cttcaatagg ttcaatgctt ggatgaagaa gcttgccact cccgccacca tctctaggta     240

gctgaagagg agcgagaaca aaatagaaaa agggttgcat tctatcctga tgcagtcaaa     300

ggagtagctt actatagaga agtctcgagc tgacacagta gaggctgctt ttggggaagc     360

caagacctct ctaaaagaag ttaagaatct ttgaagataa aggttgctca gccccaag       418